(19) Europäisches Patentamt
European Patent Office
Office européen des brevets

(11) Veröffentlichungsnummer: **0 034 259**
**B1**

(12) # EUROPÄISCHE PATENTSCHRIFT

(45) Veröffentlichungstag der Patentschrift: **12.06.85**

(51) Int. Cl.⁴: **A 61 K 37/02**, C 07 K 7/06

(21) Anmeldenummer: **81100356.5**

(22) Anmeldetag: **19.01.81**

(54) **Peptidester, Verfahren zur Herstellung derselben und diese enthaltende Arzneimittel beziehungsweise Diagnostica.**

(30) Priorität: **18.01.80 HU 1018080**

(43) Veröffentlichungstag der Anmeldung: **26.08.81 Patentblatt 81/34**

(45) Bekanntmachung des Hinweises auf die Patenterteilung: **12.06.85 Patentblatt 85/24**

(84) Benannte Vertragsstaaten: **AT BE CH DE FR GB IT LI NL SE**

(56) Entgegenhaltungen:
**DE - A - 2 831 271**
**DE - A - 2 831 534**
**US - A - 3 923 769**
**US - A - 3 923 770**
**US - A - 3 976 770**

(73) Patentinhaber: **RICHTER GEDEON VEGYESZETI GYAR R.T., Gyömröi ut 19-21, H-1475 Budapest X (HU)**

(72) Erfinder: **Nyéki, Olga, Dipl. Chem., Ungvár u. 56/g, Budapest XIV (HU)**
Erfinder: **Kisfaludy, Lajos, Dr. Dipl.-Chem.-Ing., Riadó u. 6/A, Budapest II (HU)**
Erfinder: **Kárpáti, Egon, Dr., Krisztina krt. 35, Budapest XI (HU)**
Erfinder: **Szporny, László, Dr., Szabolcska M. u. 7, Budapest XI (HU)**

(74) Vertreter: **Beszédes, Stephan G. Dr., Münchener Strasse 80a Postfach 1168, D-8060 Dachau (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue in der 8-Stellung eine Aminosäureestergruppe aufweisende Peptidester, ein Verfahren zu ihrer Herstellung und diese enthaltende Arzneimittel beziehungsweise Diagnostica, insbesondere solche mit Angiotensin-II entgegenwirkender beziehungsweise antagonisierender Wirkung.

Das erste Angiotensin-II-analogon, welches sowohl in vitro als auch in vivo als spezifischer Konkurrenzinhibitor von Angiotensin-II wirkt, wurde im Jahre 1970 beschrieben (vergleiche: G.R. Marshal und Mitarbeiter, Proc. Nat. Acad. Sci. USA, 67 [1970], 1 624; P.A. Khairallah und Mitarbeiter, J. Med. Chem. 13 [1970], 181).

Diese Beobachtung hat dann zu ausgedehnten weiteren Forschungsarbeiten Anlass gegeben, welche zur Herstellung von zahlreichen weiteren solchen Angiotensin-II entgegenwirkenden beziehungsweise antagonisierenden Angiotensin-II-analoga führten; solche Produkte boten Möglichkeiten zur Diagnostizierung und gegebenenfalls auch zur therapeutischen Behandlung von bestimmten reninabhängigen Bluthochdruck- beziehungsweise Hypertensionserscheinungen. Von den auf diese Weise hergestellten zahlreichen Angiotensin-II entgegenwirkenden beziehungsweise antagonisierenden Verbindungen ist bereits das L-Sarkosyl-L-arginyl-L-valyl-L-tyrosyl-L-isoleucyl-L-histidyl-L-prolyl-L-alanin ([$Sar^1$,$Ala^8$]-angiotensin-II) unter der Bezeichnung Saralasin in den Verkehr gebracht worden (D. T. Pals und Mitarbeiter: Circ. Res. 29 [1971], 673); dieses Produkt hat sich im Laufe der klinischen Untersuchungen als zur Diagnostizierung (G. Bönner und Mitarbeiter: Dtsch. Med. Wschr. 104 [1979], 432) beziehungsweise zur therapeutischen Behandlung (J.L. Marx: Science 194 [1976], 821) von Bluthochdruck beziehungsweise Hypertensionen verschiedenen Ursprungs geeignet erwiesen. Neuerdings wurde festgestellt, dass die Angiotensin-II-antagonisten auch zur Behandlung der als Folgeerscheinung des renovaskularen Bluthochdruckes auftretenden Herzinsuffizienz geeignet sind (H. Gavras und Mitarbeiter: JAMA 238 [1977], 880).

Die Untersuchung der Zusammenhänge zwischen der Struktur und der biologischen Wirkung der bekannten Angiotensin-II-analoga hat auch nützliche Informationen zur Klärung der agonistischen und antagonistischen Wirkungen geliefert. Im Vordergrund der gegenwärtigen Forschungen steht die Herstellung von solchen Antagonisten, welche längere biologische Halbwertszeiten und keine unerwünschten Nebenwirkungen, wie anfängliche agonistische Wirkungen nach der Verabreichung, zeigen.

Ferner sind aus der deutschen Offenlegungsschrift 2 831 271 Peptide der allgemeinen Formel

X-Arg-Val-Tyr-Ile-His-Pro-Y,

worin

X den Rest einer in der α-Stellung eine Hydroxygruppe enthaltenden aliphatischen Carbonsäure, insbesondere eine Hydroxyacetyl- oder α-Hydroxypropionylgruppe, und

Y den Rest einer aliphatischen α-Aminocarbonsäure, insbesondere eine Leucyl-, Isoleucyl-, Alanyl- oder Threonylgruppe,

bedeuten, und ihre Säureadditionssalze und Komplexe sowie ihre pharmakologischen, insbesondere Angiotensin-II entgegenwirkenden, Wirkungen bekannt. Ausserdem wird im Schritt 3 des Beispieles 6 der deutschen Offenlegungsschrift 2 831 271 von einem an verschiedenen Carbonsäureeinheiten geschützten Octapeptidester der abgekürzten Bezeichnung Z-OGly-Arg($NO_2$)-Val-Tyr(Bzl)-Ile-His(Dnp)-Pro-Thr(ME)-OMe ausgegangen und auch für den im selben Schritt desselben Beispieles auf Seite 35, Zeile 17 bis 18 der deutschen Offenlegungsschrift 2 831 271 stehenden als Zwischenprodukt erhaltenen Octapeptidester mit der Kurzbezeichnung Z-OGly-Arg($NO_2$)-Val-Tyr(Bzl)-Ile-His-Pro-Thr(Me)-OMe sind solche Schutzgruppen angegeben. Dieser wird dann mit einer Natriumhydroxydlösung behandelt, worauf gemäss Seite 35, Zeile 26 bis 27 der deutschen Offenlegungsschrift 2 831 271 ein an der C-terminalen Aminosäure freies Peptid, welches also keine Methylestergruppe mehr aufweist, erhalten wird. Es ist auch noch zu bemerken, dass für die Zwischenprodukte des Schrittes 3 des Beispieles 6 der deutschen Offenlegungsschrift 2 831 271 keine pharmakologische Wirkung angegeben ist.

Weiterhin sind in der deutschen Offenlegungsschrift 2 831 534 Peptide der allgemeinen Formel

X-Arg-Val-Tyr-Ile-His-Pro-Y,

worin

X den Rest einer in der α-Stellung eine Aminooxygruppe enthaltenden aliphatischen Carbonsäure, insbesondere eine Aminooxyacetyl- oder α-Aminooxypropionylgruppe, und

Y den Rest einer aliphatischen α-Aminocarbonsäure, insbesondere eine Leucyl-, Isoleucyl-, Alanyl- oder Threonylgruppe,

bedeuten, und ihre Säureadditionssalze und Komplexe sowie ihre pharmakologischen, insbesondere Angiotensin-II entgegenwirkenden, Wirkungen beschrieben.

Ausserdem ist aus der US-Patentschrift 3 923 769 das Octapeptid L-(N-methyl)Ile-L-Arg-L-Val-L-Tyr-L-Ile-L-His-L-Pro-L-Ile bekannt, wobei seine pharmakologische Wirksamkeit als Angiotensin-II entgegenwirkend ohne Vergleichsversuchsergebnisse angegeben ist.

Ferner ist in der US-Patentschrift 3 923 770 das Octapeptid Dimethylglycyl-L-Arg-L-Val-L-Tyr-L-Ile-L-His-L-Pro-L-Ile beschrieben, wobei seine pharmakologische Wirksamkeit als Angiotensin-II entgegenwirkend wiederum ohne Vergleichsversuchsergebnisse angegeben ist.

Ausserdem betrifft die US-Patentschrift 3 976 770 das Octapeptid Sar-L-Arg-L-Val-L-Tyr-L-Ile-L-His-L-Pro-(OMe)Thr, wobei auch in dieser Druckschrift die pharmakologische Wirksamkeit dieser Verbindung als Angiotensin-II entgegenwirkend ohne Vergleichsversuchsergebnisse angegeben ist.

Der Erfindung liegt die Aufgabe zugrunde, neue Peptidderivate mit überlegenen pharmakologischen Wirkungen, insbesondere Angiotensin-II entgegenwirkenden beziehungsweise antagonisierenden Wirkungen, ohne unerwünschte Nebenwirkungen, ein Verfahren zur Herstellung derselben und diese Verbindungen enthaltende Arzneimittel zu schaffen.

Das Obige wurde überraschenderweise durch die Erfindung erreicht.

Es wurde nämlich überraschenderweise festgestellt, dass wirksame Konkurrenzinhibitoren beziehungsweise kompetitive Inhibitoren von Angiotensin-II durch Ersatz der Phenylalanineinheit in der 8-Stellung des Moleküles von Angiotensin-II durch eine aliphatische Aminosäureeinheit und Einbauen einer N-Methylaminosäureeinheit oder einer aliphatischen α-Aminooxy- beziehungsweise α-Hydroxycarbonsäureeinheit in die 1-Stellung des Moleküles sowie ferner Verestern der C-endständigen Carboxylgruppe des Moleküls mit einer Alkylgruppe mit 1 bis 5 Kohlenstoffatomen erzielt werden können.

Gegenstand der Erfindung sind daher Peptidester der allgemeinen Formel

X-Arg-Val-Tyr-Ile-His-Pro-Y-O-A I,

worin

X für einen Acylrest einer N-Methylaminosäure oder einer in der α-Stellung eine Aminooxygruppe oder eine Hydroxygruppe aufweisenden aliphatischen Carbonsäure, jeweils in der L-Konfiguration im Falle eines asymmetrischen Kohlenstoffatomes, steht,

Arg einen Rest von L-Arginin bedeutet,

Val einen Rest von L-Valin darstellt,

Tyr für einen Rest von L-Tyrosin steht,

Ile einen Rest von L-Isoleucin bedeutet,

His einen Rest von L-Histidin darstellt,

Pro für einen Rest von L-Prolin steht,

Y einen Rest einer aliphatischen Aminosäure jeweils in der L-Konfiguration im Falle eines asymmetrischen Kohlenstoffatoms, bedeutet und

A einen Alkylrest mit 1 bis 5 Kohlenstoffatom(en) darstellt,

sowie ihre Säureadditionssalze und pharmazeutisch brauchbaren Komplexe.

Von den in der deutschen Offenlegungsschrift 2 831 271 beanspruchten Octapeptiden unterscheiden sich alle erfindungsgemässen Verbindungen darin, dass sie an ihrer Aminosäureeinheit in der 8-Stellung durch einen Alkylrest verestert sind, also Peptidester sind. Auch von den Zwischenprodukten des Beispieles 6, Schritt 3 der deutschen Offenlegungsschrift 2 831 271 sind die erfindungsgemässen Verbindungen verschieden, indem sie keine Schutzgruppen aufweisen beziehungsweise an der C-terminalen Aminosäure nicht frei, sondern verestert sind. Soweit in den erfindungsgemässen Peptidestern X für einen Acylrest einer N-Methylaminosäure oder einer in der α-Stellung eine Aminooxygruppe aufweisenden aliphatischen Carbonsäure steht, kommt dies als zusätzlicher Unterschied gegenüber den Verbindungen der deutschen Offenlegungsschrift 2 831 271 hinzu.

Von den Octapeptiden der deutschen Offenlegungsschrift 2 831 534 unterscheiden sich sämtliche erfindungsgemässen Verbindungen darin, dass ihre Aminosäureeinheit in der 8-Stellung durch einen Alkylrest verestert ist, sie also Peptidester sind. Als zusätzlicher Unterschied der erfindungsgemässen Peptidester, bei welchen X für einen Acylrest einer N-Methylaminosäure oder einer in der α-Stellung eine Hydroxygruppe aufweisenden aliphatischen Carbonsäure steht, gegenüber den Verbindungen der deutschen Offenlegungsschrift 2 831 534 kommt noch diese Bedeutung von X hinzu.

Sämtliche erfindungsgemässen Verbindungen unterscheiden sich auch von der Verbindung der US-Patentschrift 3 923 769 darin, dass bei ihnen die Aminosäureeinheit in der 8-Stellung durch einen Alkylrest verestert ist, sie also Peptidester sind. Die erfindungsgemässen Verbindungen, bei welchen X für einen vom L-(N-Methyl)-isoleucylrest verschiedenen Acylrest einer N-Methylaminosäure oder für einen Acylrest einer in der α-Stellung eine Aminooxygruppe oder eine Hydroxygruppe aufweisenden aliphatischen Carbonsäure steht und/oder Y einen von einem Rest von L-Isoleucin verschiedenen Rest einer aliphatischen Aminosäure bedeutet, unterscheiden sich von der Verbindung der US-Patentschrift 3 923 769 noch zusätzlich durch diese Bedeutung(en).

Auch von der Verbindung der US-Patentschrift 3 923 770 unterscheiden sich sämtliche erfindungsgemässen Verbindungen darin, dass bei ihnen die Aminosäureeinheit in der 8-Stellung durch einen Alkylrest verestert ist, sie also Peptidester sind. Darüberhinaus können die erfindungsgemässen Peptidester in der 1-Stellung (für X) keinen Acylrest der N,N-Dimethylessigsäure, die für die 1-Stellung der Verbindung der genannten Druckschrift zwingend vorgeschrieben ist, aufweisen. Bei den erfindungsgemässen Verbindungen, bei welchen Y von einem Rest von L-Isoleucin verschieden ist, kommt gegenüber der Verbindung der US-Patentschrift 3 923 770 noch der Unterschied hinsichtlich dieser Bedeutung hinzu.

Auch vom Octapeptid der US-Patentschrift 3 976 770 unterscheiden sich sämtliche erfindungsgemässen Verbindungen darin, dass ihre Aminosäureeinheit in der 8-Stellung durch einen Alkylrest verestert ist. Die erfindungsgemässen Verbindungen, bei welchen X für einen vom Sarkosylrest verschiedenen Acylrest einer N-Methylaminosäure oder einen Acylrest einer in der α-Stellung eine Aminooxygruppe oder eine Hydroxygruppe aufweisenden aliphatischen Carbonsäure steht und/oder Y einen von einem Rest von L-Methylthreonin verschiedenen Rest einer aliphatischen Aminosäure bedeutet, unterscheiden sich von der Verbindung der US-Patentschrift 3 976 770 noch zusätzlich hinsichtlich dieser Bedeutung(en).

Auf Grund dieser Unterschiede stellen die erfindungsgemässen Peptide eine neue Klasse von Angiotensin-II entgegenwirkenden Verbindungen, welche bisher nicht beschrieben und somit auch pharmakologisch nicht untersucht wurden, dar. Gegenüber den US-Patentschriften 3 923 769, 3 923 770 und 3 976 770 ist zu bemerken, dass in diesen nicht einmal ein Vergleich der in ihnen beschriebenen Peptide mit dem damals schon bekannten und angewandten L-Sarkosyl-L-arginyl-L-valyl-L-tyrosyl-L-isoleucyl-L-histidyl-L-prolyl-L-alanin [Saralasin] gemacht worden ist. Daraus aber, dass diese bekannten Octapeptide in den seitdem abgelaufenen 5 bis 6 Jahren am internationalen Markt nicht durchdringen konnten, ist zu schliessen, dass sie mit dem L-Sarkosyl-L-arginyl-L-valyl-L-tyrosyl-L-isoleucyl-L-histidyl-L-prolyl-L-alanin [Saralasin] nicht zu konkurrieren vermochten.

Vorzugsweise ist der Acylrest einer N-Methylaminosäure, für den X stehen kann, der Sarkosylrest.

Es ist auch bevorzugt, dass der Alkylrest, für den A steht, ein solcher mit 1 oder 2, ganz besonders 1, Kohlenstoffatom(en) ist.

Ferner ist es bevorzugt, dass der Rest einer Aminosäure, für den Y steht, ein solcher von L-Isoleucin, L-Threonin, L-Methylthreonin oder L-Alanin ist.

Besonders bevorzugte erfindungsgemässe Verbindungen sind

L-Sarkosyl-L-arginyl-L-valyl-L-tyrosyl-L-isoleucyl-L-histidyl-L-prolyl-L-isoleucinmethylester,

L-α-Aminooxypropionyl-L-arginyl-L-valyl-L-tyrosyl-L-isoleucyl-L-histidyl-L-prolyl-L-isoleucinmethylester,

L-Sarkosyl-L-arginyl-L-valyl-L-tyrosyl-L-isoleucyl-L-histidyl-L-prolyl-L-methylthreoninmethylester,

Hydroxyacetyl-L-arginyl-L-valyl-L-tyrosyl-L-isoleucyl-L-histidyl-L-prolyl-L-isoleucinmethylester,

Hydroxyacetyl-L-arginyl-L-valyl-L-tyrosyl-L-isoleucyl-L-histidyl-L-prolyl-L-methylthreoninmethylester,

Hydroxyacetyl-L-arginyl-L-valyl-L-tyrosyl-L-isoleucyl-L-histidyl-L-prolyl-L-threoninmethylester und

L-Sarkosyl-L-arginyl-L-valyl-L-tyrosyl-L-isoleucyl-L-histidyl-L-prolyl-L-alaninmethylester.

Unter pharmazeutisch brauchbaren Komplexen der erfindungsgemässen Peptidester der allgemeinen Formel I sind solche mit organischen oder anorganischen Verbindungen, durch welche eine verzögerte Wirkung beziehungsweise Retardwirkung der Peptidester gewährleistet ist, zu verstehen. Als organische Verbindungen sind zum Beispiel Gelatine, Carboxymethylcellulosen, Alginsäureester, Poly-(phloretinphosphate), Aminosäurepolymere und -copolymere geeignet. Als anorganische Verbindungen kommen beispielsweise Hydroxyde beziehungsweise schwerlösliche Salze, wie Phosphate, von Metallen, insbesondere von Zink, in Frage.

Gegenstand der Erfindung ist auch ein Verfahren zur Herstellung der erfindungsgemässen Verbindungen, welches dadurch gekennzeichnet ist, dass in in der Peptidchemie an sich bekannter Weise ein Alkylester einer passenden aliphatischen Aminosäure mit 1 bis 5 Kohlenstoffatomen im Alkylteil mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat desselben kondensiert wird und gegebenenfalls mit dem erhaltenen am endständigen Stickstoffatom geschützten Peptidzwischenprodukt und dem beziehungsweise den durch etwaige weitere derartige Kondensationen erhaltenen am endständigen Stickstoffatom geschützten weiteren Peptidzwischenprodukt(en) nach in an sich bekannter Weise erfolgendem Entfernen der Schutzgruppe des endständigen Stickstoffatomes eine weitere beziehungsweise weitere Kondensation(en) mit beziehungsweise jeweils mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat desselben vorgenommen wird beziehungsweise werden, wobei so viele Kondensationen durchgeführt werden, wie es zum Einbau aller gewünschten Aminosäureeinheiten erforderlich ist, sowie danach in an sich bekannter Weise vom erhaltenen geschützten Peptidesterderivat die Schutzgruppe des endständigen Stickstoffatoms und die etwaige(n) Schutzgruppe(n) von anderen Stickstoffatomen beziehungsweise Sauerstoffatomen sowie gegebenenfalls die Aminogruppe der gegebenenfalls vorliegenden Aminooxygruppe selektiv schrittweise oder in einem Schritt entfernt wird beziehungsweise werden, worauf gegebenenfalls in an sich bekannter Weise der erhaltene Peptidester der allgemeinen Formel I in ein Säureadditionssalz oder einen Komplex desselben überführt wird beziehungsweise gegebenenfalls das erhaltene Säureadditionssalz des Peptidesters der allgemeinen Formel I in ein anderes Säureadditionssalz oder in den Peptidester der allgemeinen Formel I überführt wird.

Vorzugsweise werden durch die Kondensation(en) als geschützte Peptidesterderivate solche der allgemeinen Formel

B-X-Arg[C]-Val-Tyr[D]-Ile-His[E]-
Pro-Y-O-A II,

worin

B für eine durch Acidolyse oder katalytische Hydrogenolyse entfernbare Schutzgruppe, insbesondere eine Benzyloxycarbonyl- oder tert.-Butyloxycarbonylgruppe, steht,

C eine Schutzgruppe zum zeitweiligen Schutz der Guanidinogruppe des Restes von von Arginin, insbesondere eine Nitro- oder p-Toluolsulfonylgruppe (Tosylgruppe), bedeutet,

D eine Schutzgruppe zum zeitweiligen Schutz der aromatischen Hydroxygruppe des Restes von Tyrosin, insbesondere eine, gegebenenfalls substituierte, Benzylgruppe, darstellt,

E für eine Schutzgruppe zum zeitweiligen Schutz der Imidazolgruppe des Restes von Histidin, insbesondere eine Dinitrophenylgruppe, steht und

X, Arg, Val, Tyr, Ile, His, Pro, Y und A wie oben festgelegt sind, aufgebaut.

Im erfindungsgemässen Verfahren können die Kondensationen zum Aufbau des geschützten Peptidesterderivates, wie des der allgemeinen Formel II, nach beliebigen in der Peptidchemie üblichen Verfahrensweisen, zum Beispiel nach der in der ungarischen Patentschrift 168 431 beschriebenen Verfahrensweise durch Acylieren der passenden Aminosäurealkylester mit Pentafluorphenylestern von an der N-endständigen Aminogruppe geschützten Aminosäuren beziehungsweise Peptiden durchgeführt werden. Dabei sind zum Schutz der seitenfunktionellen Gruppen solche Schutzgruppen, welche unter den Bedingungen der nach dem Acylieren durchzuführenden Entfernung der N-Schutzgruppe, beispielsweise durch Acidolyse, stabil sind, zu verwenden.

Nach einer vorteilhaften Ausführungsform des erfindungsgemässen Verfahrens werden also die geschützten Peptidesterderivate, wie die der allgemeinen Formel II, schrittweise aus den einzelnen Aminosäuren aufgebaut. Nach einer anderen Ausführungsform des erfindungsgemässen Verfahrens kann aber auch ein bereits hergestelltes entsprechend geschütztes Teilpeptid auf einmal mit einem anderen ebenfalls bereits hergestelltem Teilpeptid acyliert werden. Diese Verfahrensweise kann zum Beispiel bei der Herstellung der in der 1-Stellung eine α-Aminooxyacylgruppe enthaltenden Peptide vorteilhaft angewandt werden. Zum zeitweiligen Schutz der Aminogruppen der einzelnen Aminosäuren beziehungsweise Aminosäurederivate beziehungsweise Teilpeptide sind bei beiden Ausführungsformen solche Schutzgruppen zu verwenden, welche dann durch bekannte Verfahrensweisen, wie durch Acidolyse, leicht entfernt werden können; besonders die tert.-Butyloxycarbonylgruppe ist für solche Zwekke vorteilhaft anwendbar.

Nach der Beendigung des Aufbaues des geschützten Peptidesterderivates, wie des der allgemeinen Formel II, werden dann wie bereits erwähnt dessen Schutzgruppen abgespalten. Dabei wird zweckmässig in der Weise vorgegangen, dass zunächst die Dinitrophenylschutzgruppe des Restes von Histidin, soweit eine solche vorhanden ist, durch Thiolyse, und zwar vorzugsweise durch Behandlung mit 2-Mercaptoäthanol, abgespalten wird. Die übrigen vorliegenden Schutzgruppen können, soweit eine solche Thiolyse durchgeführt wird, nach ihr, vorteilhaft in einem Schritt, und zwar vorzugsweise durch Acidolyse, insbesondere mit Fluorwasserstoffsäure, oder katalytische Hydrogenolyse, insbesondere unter Verwendung von Palladium/Aktivkohle als Katalysator, entfernt werden.

Nach einer vorteilhaften Ausführungsform des erfindungsgemässen Verfahrens werden zur Herstellung von Peptidestern der allgemeinen Formel I, bei welchen X für einen Acylrest einer in der α-Stellung eine Aminooxygruppe aufweisenden aliphatischen Carbonsäure steht, durch die Kondensation(en) die entsprechenden geschützten Peptidester der allgemeinen Formel II, bei welchen X ebenfalls für einen Acylrest einer in der α-Stellung eine Aminooxygruppe aufweisenden aliphatischen Carbonsäure steht, aufgebaut und das Abspalten der Schutzgruppe, für welche B steht, sowie gegebenenfalls der anderen durch Acidolyse abspaltbaren Schutzgruppen von ihnen durch Acidolyse, insbesondere durch Behandeln der, zweckmässig von der Dinitrophenylgruppe befreiten, geschützten Peptidesterderivate mit Fluorwasserstoffsäure, durchgeführt. Die katalytische Hydrierung kommt hierfür deswegen nicht in Frage, weil durch diese gleichzeitig auch der Acylrest der in der α-Stellung eine Aminooxygruppe aufweisenden aliphatischen Carbonsäure, für den X steht, unter Abspalten der Aminogruppe in den entsprechenden Acylrest der in der α-Stellung eine Hydroxygruppe aufweisenden sonst gleichen aliphatischen Carbonsäure überführt würde.

Nach einer anderen vorteilhaften Ausführungsform des erfindungsgemässen Verfahrens werden zur Herstellung von Peptidestern der allgemeinen Formel I, bei welchen X für einen Acylrest einer in der α-Stellung eine Hydroxygruppe aufweisenden aliphatischen Carbonsäure steht, durch die Kondensation(en) die entsprechenden Peptidester der allgemeinen Formel II, bei welchen jedoch X für einen Acylrest einer in der α-Stellung eine Aminooxygruppe aufweisenden aliphatischen Carbonsäure steht, aufgebaut und im Gegensatz zur vorstehend genannten Ausführungsform des erfindungsgemässen Verfahrens das Abspalten der Schutzgruppe für welche B steht, sowie gegebenenfalls der anderen durch katalytische Hydrogenolyse abspaltbaren Schutzgruppen von ihnen durch katalytische Hydrierung unter gleichzeitiger Abspaltung der Aminogruppe der genannten α-Aminooxygruppe durchgeführt.

Das gegebenenfalls erfolgende Überführen der erfindungsgemässen Peptidester der allgemeinen Formel I in Säureadditionssalze beziehungsweise Komplexe kann nach an sich bekannten Verfahrensweisen durchgeführt werden.

Die erfindungsgemässen Peptidester können durch bekannte Verfahrensweisen, zum Beispiel durch Ionenaustauschchromatographie an Carboxymethylcellulose, gereinigt werden. Sie werden zweckmässig in Form von lyophilisierten Pulvern hergestellt, welche sich sehr gut zur Zubereitung von verschiedenen Arzneimittelpräparaten eignen.

Ferner sind erfindungsgemäss Arzneimittel beziehungsweise Diagnostica, welche 1 oder mehr erfindungsgemässe Verbindungen als Wirkstoff beziehungsweise Wirkstoffe, zweckmässigerweise zusammen mit üblichen pharmazeutischen Konfektionierungsmitteln, enthalten, vorgesehen.

Die erfindungsgemässen Verbindungen haben nämlich wie bereits erwähnt wertvolle pharmakologische Wirkungen, insbesondere Angiotensin-II entgegenwirkende beziehungsweise antagonisierende Wirkungen.

Sie verringern in erheblichem Masse den durch Angiotensin-II herbeigeführten hohen Blutdruck und haben dabei den grossen Vorteil, dass sie diese Wirkung auch bei subkutaner Verabreichung zeigen.

Die Angiotensin-II entgegenwirkende beziehungsweise antagonistische Wirkung von erfindungsgemässen Verbindungen und der anerkannt gut wirksamen Vergleichssubstanz L-Sarkosyl-L-arginyl-L-valyl-L-tyrosyl-L-isoleucyl-L-histidyl-L-prolyl-L-alanin ([$Sar^1$,$Ala^8$]-angiotensin-II){Saralasin} gleicher Wirkungsrichtung wurde an narkotisierten männlichen Katzen geprüft. Bei den Untersuchungen wurden die Vagusnerven der Tiere an beiden Seiten des Halses durchgeschnitten und nach der Blockierung der Ganglien wurde den Tieren Hypertensin (Ciba) durch Infusion mit einer Geschwindigkeit von 0,5 µg/kg/Minute verabreicht. Nachdem sich der erhöhte Blutdruck stabilisiert hatte, wurde die zu untersuchende Verbindung in physiologischer Kochsalzlösung beziehungsweise in einer Trägerstoffe enthaltenden Lösung intravenös oder subkutan verabreicht. Die darauf eingetretene Blutdrucksenkung in mm Hg wurde in Prozenten des vor der Behandlung gemessenen Blutdruckanfangswertes registriert. Die statistische Auswertung der Messergebnisse erfolgte auf Grund des Student'schen 1-Proben-«t»-Versuches. Als Wirkungsdauer der einzelnen Wirkstoffe wurde die bis zur letzten noch signifikanten Blutdrucksenkung ($p = 5\%$) verstrichene Zeit zugrundegelegt. Die so erhaltenen Versuchsergebnisse sind in der folgenden Tabelle zusammengestellt.

Aus der obigen Tabelle geht hervor, dass die erfindungsgemässen Peptidester in überlegenen blutdrucksenkenden Wirkungen sich äussernde überlegene Angiotensin-II entgegenwirkende beziehungsweise antagonisierende Wirkungen haben, was ein Zeichen dafür ist, dass die Ladung der C-endständigen Carboxylgruppe keine notwendige Bedingung für das Bestehen dieser Wirkung ist. Das Mass der Wirkung ist auch bei subkutaner Verabreichung dieser Verbindungen sehr hoch; in Trägerstoffe enthaltenden Lösungen werden in zahlreichen Fällen Wirkungsdauern von mehreren Stunden erreicht.

Die erfindungsgemässen Peptidester einschliesslich ihrer Säureadditionssalze und Komplexe können in Form von üblichen Arzneimittelpräparaten vorliegen und in der Therapie angewandt werden. Diese Arzneimittelpräparate enthalten die erfindungsgemässen Verbindungen in Verbindung mit zur enteralen oder parenteralen Verabreichung geeigneten anorganischen oder organischen Träger- und/oder Hilfsstoffen. Sie können zum Beispiel in Form von festen Lyophilisaten, welche als Trägerstoffe pharmazeutisch brauchbare und mit den erfindungsgemässen Peptidestern nicht reagierende feste Stoffe, wie Kohlenhydrate, enthalten, sowie ferner in Form von neben den erfindungsgemässen Peptidesterwirkstoffen gegebenenfalls auch konservierend oder stabilisierend wirkende Hilfsstoffe enthaltenden verdünnten oder konzentrierten Suspensionen oder Emulsionen vorliegen. Die Zubereitung dieser Arzneimittelpräparateformen kann nach üblichen pharmazeutischen Verfahrensweisen erfolgen.

Die erfindungsgemässen Arzneimittel können zum differenzierenden Diagnostizieren von hohem Blutdruck verschiedenen Ursprunges sowie ferner in der Therapie beispielsweise zum Normalisieren von von den Nieren herrührendem hohem Blutdruck beziehungsweise Hypertensionen renalen Ursprunges, bei Bluthochdruck- beziehungsweise Hypertensionskrisen und in Fällen von sekundärer Herzinsuffizienz eingesetzt werden.

Die erfindungsgemässen Arzneimittelpräparate liegen zweckmässig in Form von 1 bis 10 mg Wirkstoff enthaltenden Injektionslösungen vor. Die tägliche Dosis der erfindungsgemässen Peptidesterwirkstoffe beträgt bei erwachsenen Patienten zweckmässig 1 bis 10 mg. Diese Menge wird vorzugsweise auf einmal intravenös, subkutan, intramuskulär oder mit langsamer intravenöser Infusion verabreicht.

Die Erfindung wird an Hand der folgenden Beispiele näher erläutert.

Die in Beispielen verwendeten Abkürzungen von Aminosäuren und Schutzgruppen entsprechen den im Schrifttum allgemein üblichen Abkürzungen (vgl. J. Biol. Chem. 247 [1972], 977. Ferner wurden noch die folgenden Abkürzungen verwendet:

HOAA = Hydroxyessigsäure,
OAla = α-Aminooxypropionsäure,
OGly = Aminooxyessigsäure und
Pfp = Pentafluorphenyl.

Bei der Herstellung der verschiedenen Verbindungen wurde das Eindampfen der Lösungen jeweils in einem Vakuumeindampfer vom Typ «Rotavapor» durchgeführt. Zur Bestimmung von Schmelzpunkten wurde ein Schmelzpunktmessgerät nach Dr. Totoli (Büchi) verwendet. Die Dünnschichtchromatogramme wurden auf Silicagel-

Tabelle
Wirkung auf den Blutdruck

| Verbindung | | Verabreichung und deren Wirkung | | | | | | | | | | | | | | | |
|---|---|---|---|---|---|---|---|---|---|---|---|---|---|---|---|---|---|
| Bei-spiel | Bezeichnung | intravenös | | | | subkutan in physiologischer Kochsalzlösung | | | | subkutan in Carboxymethyl-celluloselösung | | | | subkutan in Gelatinelösung | | | |
| | | Dosis in µg/kg | Zahl der Versuche | Blutdrucksenkung in % | Zeitdauer in Minuten | Dosis in µg/kg | Zahl der Versuche | Blutdrucksenkung in % | Zeitdauer in Minuten | Dosis in µg/kg | Zahl der Versuche | Blutdrucksenkung in % | Zeitdauer in Minuten | Dosis in µg/kg | Zahl der Versuche | Blutdrucksenkung in % | Zeitdauer in Minuten |
| 1 | L-Sarkosyl-L-arginyl-L-valyl-L-tyrosyl-L-isoleucyl-L-histidyl-L-prolyl-L-isoleucinmethylester {[$Sar^1$,Ile-$OMe^8$]-Ang-II} | 20<br>40 | 8<br>7 | 21<br>20 | 25<br>38 | 50<br>100 | 5<br>5 | 8<br>25 | 180<br>240 | 100 | 10 | 17 | 200 | 100<br>200 | 8<br>5 | 5<br>23 | 15<br>300 |
| 7 | L-Sarkosyl-L-arginyl-L-valyl-L-tyrosyl-L-isoleucyl-L-histidyl-L-prolyl-L-alaninmethylester {[$Sar^1$,Ala-$OMe^8$]-Ang-II} | | | | | 50 | 7 | 20 | 45 | 50 | 6 | 11 | 22 | | | | |
| 2 | Hydroxyacetyl-L-arginyl-L-valyl-L-tyrosyl-L-isoleucyl-L-histidyl-L-prolyl-L-isoleucinmethylester {[$HOAA^1$,Ile-$OMe^8$]-Ang-II} | 20 | 4 | 27 | 42 | | | | | | | | | | | | |
| Vergleichs-substanz | L-Sarkosyl-L-arginyl-L-valyl-L-tyrosyl-L-isoleucyl-L-histidyl-L-prolyl-L-alanin {Saralasin} | 10 | 5 | 32 | 15 | 100<br>200 | 6<br>5 | 29<br>24 | 60<br>45 | 100<br>200 | 5<br>7 | 23<br>28 | 45<br>90 | 200 | 4 | 23 | keine Dauerwirkung |

platten «Kieselgel G nach Stahl» (Merck) hergestellt. Zum Entwickeln der Chromatogramme wurden die folgenden Lösungsmittelgemische verwendet:

| Lösungsmittelgemisch | Volumverhältnis der Lösungsmittel beziehungsweise des vor dem Schrägstrich stehenden Lösungsmittels zum nach dem Schrägstrich stehenden Lösungsmittelgemisches |
|---|---|
| 1. Äthylacetat: [Pyridin/Essigsäure/Wasser im Volumverhältnis von 20:6:11] | = 98:2 |
| 2. Äthylacetat: [Pyridin/Essigsäure/Wasser im Volumverhältnis von 20:6:11] | = 95:5 |
| 3. Äthylacetat: [Pyridin/Essigsäure/Wasser im Volumverhältnis von 20:6:11] | = 90:10 |
| 4. Äthylacetat: [Pyridin/Essigsäure/Wasser im Volumverhältnis von 20:6:11] | = 80:20 |
| 5. Äthylacetat: [Pyridin/Essigsäure/Wasser im Volumverhältnis von 20:6:11] | = 70:30 |
| 6. Äthylacetat: [Pyridin/Essigsäure/Wasser im Volumverhältnis von 20:6:11] | = 60:40 |
| 7. n-Butanol : Essigsäure : Wasser | = 4:1:5 |
| 8. n-Butanol : Essigsäure : Pyridin : Wasser | = 30:6:20:24 |
| 9. n-Butanol : Äthylacetat : Essigsäure : Wasser | = 1:1:1:1 |
| 10. Äthylacetat: [Pyridin/Essigsäure/Wasser im Volumverhältnis von 20:6:11] | = 75:25 |

Das Entwickeln der Flecke erfolgte mit Ninhydrin beziehungsweise mit Chlortolidin/Kaliumjodid.

Zur Reinigung der Endprodukte wurde die folgende allgemeine Verfahrensweise angewandt:

0,5 g freier Peptidester wurde in 4 $cm^3$ einer 0,01 m Ammoniumacetatlösung gelöst und dann wurde diese Lösung auf eine 0,5 l Carboxymethylcellulose (CMC-52) enthaltende und vorher mit der obigen Ammoniumacetatpufferlösung ins Gleichgewicht gebrachte Säule geschichtet. Die Säule wurde dann durch Gradienteluieren mit 1,5 l einer 0,01 m Ammoniumacetatlösung und 1,5 l einer 0,4 m Ammoniumacetatlösung, die durch einen Gradientmischer zugeführt wurden, eluiert. Die Strömungsgeschwindigkeit war 25 $cm^3$/Stunde; es wurden Fraktionen von je 10 $cm^3$ aufgefangen. Das von der Säule herabfliessende Eluat wurde mit Hilfe eines Gerätes vom Typ «LKB Uvicord-II» (LKB-Produkter AB, Schweden) laufend registriert und dann wurde die auf Grund der erhaltenen Kurve ermittelte Hauptfraktion in einer Lyophilisiereinrichtung vom Typ Leybold-Heraeus lyophilisiert. Erforderlichenfalls wurde das erhaltene Produkt ebenfalls unter Anwendung des Gradienteluierens erneut chromatographiert.

Bei der Aminosäureanalyse bedeuten die Werte in eckigen Klammern jeweils die theoretischen Werte.

$E_{Glu}$ bedeutet die auf Glutaminsäure bezogene elektrophoretische Beweglichkeit.

Beispiel 1
L-Sarkosyl-L-arginyl-L-valyl-L-tyrosyl-L-isoleucyl-L-histidyl-L-prolyl-L-isoleucinmethylester
{[Sarcosin$^1$,Isoleucin-methylester$^8$]-angiotensin-II}

1. Stufe
[N-(tert.-Butyloxycarbonyl)-N'-(nitro)]-L-arginyl-L-valyl-[O-(benzyl)]-L-tyrosyl-L-isoleucyl-[N-(dinitrophenyl)]-L-histidyl-L-prolyl-L-isoleucinmethylester
{Boc-Arg[$NO_2$]-Val-Tyr[Bzl]-Ile-His[Dnp]-Pro-Ile-OMe}

Es wurden 1,36 g (7,5 Millimol) L-Isoleucinmethylesterhydrochlorid in 30 $cm^3$ Chloroform gelöst und mit 1,05 $cm^3$ Triäthylamin und 1,90 g (5 Millimol)
[N-(tert.-Butyloxycarbonyl)]-L-prolinpentafluorphenylester
versetzt. Das Gemisch wurde bei konstant gehaltenem pH-Wert 30 Minuten stehengelassen und dann mit Wasser, mit einer 10%igen wässrigen Citronensäurelösung und schliesslich wieder mit Wasser ausgeschüttelt, über wasserfreiem Natriumsulfat getrocknet und zur Trockne eingedampft. Der als Rückstand verbliebene geschützte Dipeptidester
[N-(tert.-Butyloxycarbonyl)]-L-prolyl-L-isoleucinmethylester

($R_f^1$-Wert = 0,8) wurde sofort in 5 $cm^3$ einer 8n Lösung von Chlorwasserstoff in Dioxan gelöst und nach 15 Minuten langem Stehen wurde die Lösung mit 20 $cm^3$ wasserfreiem Äther versetzt und eingedampft. Das als Rückstand verbliebene freie Dipeptidesterhydrochlorid L-Prolyl-L-isoleucinmethylesterhydrochlorid ($R_f^5$-Wert = 0,46) wurde sofort in 30 $cm^3$ Chloroform gelöst, der pH-Wert der Lösung wurde mit Triäthylamin auf 8 eingestellt und es wurden 4,36 g (7,5 Millimol)

[N-(tert.-Butyloxycarbonyl)-(N'-dinitrophenyl)]-L-histidinpentafluorphenylester

zugesetzt. Das Gemisch wurde 1 Stunde stehengelassen, dann mit 0,83 $cm^3$ N,N-Dimethylaminoäthylamin versetzt, 10 Minuten stehengelassen und danach mit einer mit Natriumchlorid gesättigten 10%igen Citronensäurelösung, mit einer n Salzsäure, mit einer 5%igen wässrigen Natriumbicarbonatlösung und mit Wasser in der angegebenen Reihenfolge ausgeschüttelt, über wasserfreiem Natriumsulfat getrocknet und zur Trockne eingedampft. Der als Rückstand verbliebene geschützte Tripeptidester

[N-(tert.-Butyloxycarbonyl)-(N'-dinitrophenyl)]-L-histidyl-L-prolyl-L-isoleucinmethylester

($R_f^2$-Wert = 0,45) wurde ohne Isolierung in 10 $cm^3$ einer 8n Lösung von Chlorwasserstoff in Dioxan gelöst, die Lösung wurde 15 Minuten stehengelassen und dann wurde durch Zugabe von wasserfreiem Äther das freie Tripeptidesterhydrochlorid

[N-(Dinitrophenyl)]-L-histidyl-L-prolyl-L-isoleucinmethylesterhydrochlorid

gefällt, abfiltriert und gewaschen. Dieses freie Tripeptidesterhydrochlorid

[N-(Dinitrophenyl)]-L-histidyl-L-prolyl-L-isoleucinmethylesterhydrochlorid

($R_f^5$-Wert = 0,30) wurde sofort in 15 $cm^3$ Dimethylformamid gelöst, der pH-Wert der Lösung wurde mit Triäthylamin auf 8 eingestellt und es wurden 2,4 g (6 Millimol)

[N-(tert.-Butyloxycarbonyl)]-L-isoleucinpentafluorphenylester

zugesetzt. Die Lösung wurde bei konstant gehaltenem pH-Wert 30 Minuten stehengelassen, dann wurde das Lösungsmittel abgedampft, der Rückstand wurde in Äthylacetat gelöst und die Lösung wurde mit einer 10%igen wässrigen Citronensäurelösung, mit einer n Salzsäure und schliesslich mit Wasser ausgeschüttelt, über wasserfreiem Natriumsulfat getrocknet und eingedampft. Der Rückstand wurde mit einem Gemisch von Äther und n-Hexan im Volumverhältnis von 1 : 9 verrieben und abfiltriert. Der so erhaltene geschützte Tetrapeptidester

[N-(tert.-Butyloxycarbonyl)]-L-isoleucyl-[N-(dinitrophenyl)]-L-hystidyl-L-prolyl-L-isoleucinmethylester

($R_f^2$-Wert = 0,31) wurde in 7 $cm^3$ einer 8n Lösung von Chlorwasserstoff in Dioxan gelöst, die Lösung wurde 15 Minuten stehengelassen und dann wurde durch Zugabe von wasserfreiem Äther das Produkt gefällt und abfiltriert. Das so erhaltene freie Tetrapeptidesterhydrochlorid

L-Isoleucyl-[N-(dinitrophenyl)]-L-histidyl-L-prolyl-L-isoleucinmethylesterhydrochlorid

($R_f^5$-Wert = 0,38) wurde in einem Gemisch von 20 $cm^3$ Chloroform und 10 $cm^3$ Dimethylformamid gelöst, der pH-Wert der Lösung wurde mit Triäthylamin auf 8 eingestellt und es wurden 2,96 g (5,5 Millimol)

[N-(tert.-Butyloxycarbonyl)-O-(benzyl)]-L-tyrosinpentafluorphenylester

zugegeben. Die Lösung wurde bei konstant gehaltenem pH-Wert 15 Minuten stehengelassen und dann wurde das Lösungsmittel abgedampft. Der Rückstand wurde in Äthylacetat gelöst und die Lösung wurde mit 0,22 $cm^3$ N,N-Dimethylaminoäthylamin versetzt, 10 Minuten stehengelassen, dann mit einer 10%igen wässrigen Citronensäurelösung, mit einer n Salzsäure und schliesslich mit Wasser ausgeschüttelt, über wasserfreiem Natriumsulfat getrocknet und eingedampft. Der Rückstand wurde mit wasserfreiem Äther verrieben und abfiltriert. Der erhaltene geschützte Pentapeptidester

[N-(tert.-Butyloxycarbonyl)-O-(benzyl)]-L-tyrosyl-L-isoleucyl-[N-(dinitrophenyl)]-L-histidyl-L-prolyl-L-isoleucinmethylester

($R_f^2$-Wert = 0,52) wurde in 10 $cm^3$ einer 8n Lösung von Chlorwasserstoff in Dioxan gelöst und nach 15 Minuten wurde das Produkt durch Zugabe von wasserfreiem Äther gefällt, abfiltriert und mit Äther gewaschen. Das so erhaltene freie Pentapeptidesterhydrochlorid

[O-(Benzyl)]-L-tyrosyl-L-isoleucyl-[N-(dinitrophenyl)]-L-histidyl-L-prolyl-L-isoleucinmethylesterhydrochlorid

($R_f^5$-Wert = 0,8) wurde sofort in 20 $cm^3$ Dimethylformamid gelöst, der pH-Wert der Lösung wurde mit Triäthylamin auf 8 eingestellt und es wurden 2,3 g (6 Millimol)

[N-(tert.-Butyloxycarbonyl)]-L-valinpentafluorphenylester

zugesetzt. Die erhaltene Lösung wurde bei konstant gehaltenem pH-Wert 1 Stunde stehengelassen, dann wurde das Lösungsmittel abgedampft, der Rückstand wurde in Chloroform gelöst und die Lösung wurde mit einer 10%igen wässrigen Citronensäurelösung, mit einer n Salzsäure und schliesslich mit Wasser ausgeschüttelt, über wasserfreiem Natriumsulfat getrocknet und eingedampft. Der Rückstand wurde mit wasserfreiem Äther verrieben und abfiltriert. Der erhaltene geschützte Hexapeptidester

[N-(tert.-Butyloxycarbonyl)]-L-valyl-[O-(benzyl)]-L-tyrosyl-L-isoleucyl-[N-(dinitrophenyl)]-L-histidyl-L-prolyl-L-isoleucinmethylester

($R_f^2$-Wert = 0,43) wurde sofort in einer 8n Lösung von Chlorwasserstoff in Dioxan gelöst, die Lösung wurde 15 Minuten stehengelassen und dann wurde das Produkt durch Zugabe von wasserfreiem Äther gefällt, abfiltriert und gewaschen. Das erhaltene freie Hexapeptidesterhydrochlorid

L-Valyl-[O-(benzyl)]-L-tyrosyl-L-isoleucyl-[N-(dinitrophenyl)]-L-histidyl-L-prolyl-L-isoleucinmethylesterhydrochlorid

wurde sofort in 15 $cm^3$ Dimethylformamid gelöst, der pH-Wert der Lösung wurde mit Triäthylamin auf 8 eingestellt und es wurden 2,6 g (6 Millimol)
[N-(tert.-Butyloxycarbonyl)-N'-(nitro)]-
L-argininpentafluorphenylester
zugesetzt. Die Lösung wurde bei konstant gehaltenem pH-Wert 30 Minuten stehengelassen und dann mit 45 $cm^3$ Chloroform versetzt und das Gemisch wurde mit einer n Salzsäure und mit Wasser ausgeschüttelt, über wasserfreiem Natriumsulfat getrocknet und eingedampft. Der Rückstand wurde mit Äthylacetat verrieben und abfiltriert. So wurden 3,3 g des geschützten Heptapeptidesters
[N-(tert.-Butyloxycarbonyl)-N'-(nitro)]-
L-arginyl-L-valyl-[O-(benzyl)]-L-tyrosyl-
L-isoleucyl-[N-(dinitrophenyl)]-L-
histidyl-L-prolyl-L-isoleucinmethylester
($R_f^3$-Wert = 0,37) erhalten. (Diese Menge stellt eine Ausbeute von 51% der Theorie, bezogen auf den eingesetzten
[N-(tert.-Butyloxycarbonyl)]-L-prolinpenta-
fluorphenylester,
dar, woraus sich eine durchschnittliche Ausbeute von 90% der Theorie je Schritt ergibt).

2. Stufe
[N-(Benzyloxycarbonyl)]-L-sarkosyl-
[N-(nitro)]-L-arginyl-L-valyl-[O-(benzyl)]-
L-tyrosyl-L-isoleucyl-[N-(dinitrophenyl)]-
L-histidyl-L-prolyl-L-isoleucinmethylester
{Z-Sar-Arg[$NO_2$]-Val-Tyr[Bzl]-Ile-His[Dnp]-
Pro-Ile-OMe}

Es wurden 3,3 g (2,5 Millimol) wie in der vorstehenden 1. Stufe beschrieben erhaltener
[N-(tert.-Butyloxycarbonyl)-N'-(nitro)]-L-
arginyl-L-valyl-[O-(benzyl)]-L-tyrosyl-L-
isoleucyl-[N-(dinitrophenyl)]-L-histidyl-L-prolyl-
L-isoleucinmethylester
in 15 $cm^3$ einer 8n Lösung von Chlorwasserstoff in Dioxan gelöst und die Lösung wurde 20 Minuten bei Raumtemperatur stehengelassen und dann mit wasserfreiem Äther versetzt. Das gefällte Produkt wurde abfiltriert und mit Äther gewaschen. Das erhaltene freie Heptapeptidesterhydrochlorid
[N-(nitro)]-L-arginyl-L-valyl-[O-(benzyl)]-
L-tyrosyl-L-isoleucyl-[N-(dinitrophenyl)]-
L-histidyl-L-prolyl-L-isoleucinmethylester-
hydrochlorid
($R_f^5$-Wert = 0,7) wurde sofort in 15 $cm^3$ Dimethylformamid gelöst, der pH-Wert der Lösung wurde mit Triäthylamin auf 8 eingestellt und es wurden 2,4 g (6 Millimol)
[N-(Benzyloxycarbonyl)]-L-sarkosylpentafluor-
phenylester
zugesetzt. Die Lösung wurde bei konstant gehaltenem pH-Wert 30 Minuten stehengelassen, dann wurde sie mit 45 $cm^3$ Chloroform versetzt, mit einer n Salzsäure und anschliessend mit Wasser ausgeschüttelt, über wasserfreiem Natriumsulfat getrocknet und eingedampft. Der Rückstand wurde mit Äthanol verrieben und abfiltriert. So wurden 3,17 g (89% der Theorie) des geschützten Octapeptidesters
[N-(Benzyloxycarbonyl)]-L-sarkosyl-[N-(nitro)]-
L-arginyl-L-valyl-[O-(benzyl)]-L-tyrosyl-L-
isoleucyl-[N-(dinitrophenyl)]-L-histidyl-
L-prolyl-L-isoleucinmethylester
mit einem Schmelzpunkt von 197 bis 209 °C und einem ($R_f^4$-Wert von 0,82) erhalten.

3. Stufe
L-Sarkosyl-L-arginyl-L-valyl-L-tyrosyl-L-
isoleucyl-L-histidyl-L-prolyl-L-isoleucinmethylester
{Entfernen der Schutzgruppen}

Es wurden 2,59 g (1,83 Millimol) des wie in der vorstehenden 2. Stufe beschrieben erhaltenen
[N-Benzyloxycarbonyl)]-L-sarkosyl-[N-(nitro)]-
L-arginyl-L-valyl-[O-(benzyl)]-L-tyrosyl-
L-isoleucyl-[N-(dinitrophenyl)]-L-histidyl-
L-prolyl-L-isoleucinmethylesters
in 5 $cm^3$ Dimethylformamid gelöst und mit 4,6 $cm^3$ 2-Mercaptoäthanol versetzt. Das Gemisch wurde 1,5 Stunden gerührt und dann wurde durch Zugabe von wasserfreiem Äther das Produkt gefällt und abfiltriert. Es wurden 2,0 g (87% der Theorie) des geschützten, aber keine Dinitrophenylgruppe mehr enthaltenden Octapeptidesters
[N-(Benzyloxycarbonyl)]-L-sarkosyl-[N-
(nitro)]-L-arginyl-L-valyl-[O-(benzyl)]-
L-tyrosyl-L-isoleucyl-L-histidyl-L-prolyl-
L-isoleucinmethylesters
($R_f^4$-Wert = 0,42) erhalten. Dieses Produkt wurde in 40 $cm^3$ eines Gemisches von Methanol, Essigsäure und Wasser im Volumverhältnis von 5 : 1 : 1 gelöst, die Lösung wurde mit 1,0 g von 10%igem Palladium auf Aktivkohle als Katalysator versetzt und es wurde unter kräftigem Rühren beziehungsweise Schütteln 20 Stunden lang Wasserstoffgas durch die Lösung geleitet. Nach dem Ende der Reaktion wurde der Katalysator abfiltriert und mit dem gleichen Lösungsmittelgemisch gewaschen und das mit der Waschflüssigkeit vereinigte Filtrat wurde zur Trockne eingedampft. Der Rückstand wurde mit wässrigem Äthanol versetzt und erneut zur Trockne eingedampft und dieser Arbeitsgang wurde noch einige Male wiederholt. Schliesslich wurde der Rückstand mit wasserfreiem Äther verrieben, abfiltriert und getrocknet. So wurden 1,56 g (98% der Theorie) des freien Octapeptidesters
L-Sarkosyl-L-arginyl-L-valyl-L-tyrosyl-L-
isoleucyl-L-histidyl-L-prolyl-L-isoleucinmethylester
erhalten. Dieses rohe Produkt wurde nach der vor den Beispielen beschriebenen allgemeinen Reinigungsverfahrensweise gereinigt.

Der erhaltene
L-Sarkosyl-L-arginyl-L-valyl-L-tyrosyl-L-
isoleucyl-L-histidyl-L-prolyl-L-isoleucinmethylester
hatte die folgenden charakteristischen Eigenschaften:

($R_f^7$-Wert = 0,19.
($R_f^8$-Wert = 0,59.
($R_f^9$-Wert = 0,38.
$E_{Glu}$ (pH-Wert = 1,9) = 1,00.

Aminosäureanalyse:

| | | |
|---|---|---|
| L – Prolin | = | 1,07 [1], |
| L – Valin | = | 1,1 [1], |
| L – Tyrosin | = | 0,8 [1], |

| | | |
|---|---|---|
| L – Histidin | = | 1,03 [1], |
| L – Arginin | = | 1,0 [1], |
| L – Isoleucin | = | 1,87 [2] und |
| L – Sarkosin | = | 1,0 [1]. |

Beispiel 2
Hydroxyacetyl-L-arginyl-L-valyl-L-tyrosyl-L-isoleucyl-L-histidyl-L-prolyl-L-isoleucinmethylester
{[Hydroxyacetyl[1], Isoleucin-methylester[8]]-angiotensin-II}

1. Stufe
[N-(Benzyloxycarbonyl)]-L-α-aminooxyacetyl-[N-(nitro)]-L-arginyl-L-valyl-[O-(benzyl)]-L-tyrosyl-L-isoleucyl-[N-(dinitrophenyl)]-L-histidyl-L-prolyl-L-isoleucinmethylester

Es wurden 1,5 g (0,8 Millimol) des wie im Beispiel 1, Stufe 1 beschrieben hergestellten
[N-(tert.-Butyloxycarbonyl)-N′-(nitro)]-L-arginyl-L-valyl-[O-(benzyl)]-L-tyrosyl-L-isoleucyl-[N-(dinitrophenyl)]-L-histidyl-L-prolyl-L-isoleucinmethylesters
in 6 $cm^3$ einer 8n Lösung von Chlorwasserstoff in Dioxan gelöst, die Lösung wurde 20 Minuten bei Raumtemperatur stehengelassen und dann wurde durch Zugabe von wasserfreiem Äther das Produkt gefällt und abfiltriert. Das erhaltene freie Heptapeptidesterhydrochlorid
[N-(nitro)]-L-arginyl-L-valyl-[O-(benzyl)]-L-tyrosyl-L-isoleucyl-[N-(dinitrophenyl)]-L-histidyl-L-prolyl-L-isoleucinmethylesterhydrochlorid
($R_f^5$-Wert = 0,7) wurde sofort in 10 $cm^3$ Dimethylformamid gelöst, der pH-Wert der Lösung wurde mit Triäthylamin auf 8 eingestellt und es wurde 0,46 g (1,17 Millimol)
[N-(Benzyloxycarbonyl)]-L-α-aminooxyessigsäurepentafluorphenylester
zugesetzt. Das Gemisch wurde 30 Minuten stehengelassen, dann mit 30 $cm^3$ Chloroform versetzt, mit einer 10%igen wässrigen Citronensäurelösung, mit einer n Salzsäure und schliesslich mit Wasser ausgeschüttelt, über wasserfreiem Natriumsulfat getrocknet und eingedampft. Der Rückstand wurde mit Äther verrieben und abfiltriert. So wurden 1,05 g (93% der Theorie) des geschützten Aminooxyacetylheptapeptidesters
[N-(Benzyloxycarbonyl)]-L-α-aminooxyacetyl-[N-(nitro)]-L-arginyl-L-valyl-[O-(benzyl)]-L-tyrosyl-L-isoleucyl-[N-dinitrophenyl)]-L-histidyl-L-prolyl-L-isoleucinmethylester
mit einem Schmelzpunkt von 140 bis 145 °C und einem $R_f^3$-Wert von 0,24 erhalten.

2. Stufe
Hydroxyacetyl-L-arginyl-L-valyl-L-tyrosyl-L-isoleucyl-L-histidyl-L-prolyl-L-isoleucinmethylester
{Entfernen der Schutzgruppen}

Es wurden 1,05 g (0,74 Millimol) wie in der vorstehenden 1. Stufe beschrieben erhaltener
[N-(Benzyloxycarbonyl)]-L-α-aminooxyacetyl-[N-(nitro)]-L-arginyl-L-valyl-[O-(benzyl)]-L-tyrosyl-L-isoleucyl-[N-(dinitrophenyl)]-L-histidyl-L-prolyl-L-isoleucinmethylester
in 5 $cm^3$ Dimethylformamid gelöst und mit 2,3 $cm^3$ 2-Mercaptoäthanol versetzt. Das Gemisch wurde 1,5 Stunden lang bei Raumtemperatur stehengelassen und dann wurde durch Zugabe von wasserfreiem Äther das Produkt gefällt und mit Äther gewaschen. Es wurde 0,85 g (85% der Theorie) des geschützten, aber keine Dinitrophenylgruppe mehr enthaltenden Aminooxyacetylheptapeptidesters
[N-(Benzyloxycarbonyl)]-L-α-aminooxyacetyl-[N-(nitro)]-L-arginyl-L-valyl-[O-(benzyl)]-L-tyrosyl-L-isoleucyl-L-histidyl-L-prolyl-L-isoleucinmethylester
($R_f^4$-Wert = 0,40) erhalten. 0,75 g (0,6 Millimol) dieses Produktes wurden in 20 $cm^3$ eines Gemisches von Methanol, Essigsäure und Wasser im Volumverhältnis von 5 : 1 : 1 gelöst, die Lösung wurde mit 0,8 g von 10%igem Palladium auf Aktivkohle als Katalysator versetzt und es wurde unter kräftigem Rühren beziehungsweise Schütteln 18 Stunden lang Wasserstoffgas durch das Gemisch geleitet. Nach dem Ende der Reaktion wurde der Katalysator abfiltriert und mit demselben Lösungsmittelgemisch gewaschen und das mit der Waschflüssigkeit vereinigte Filtrat wurde zur Trockne eingedampft. Der Rückstand wurde mit wässrigem Äthanol mehrmals zur Trockne eingedampft, dann mit wasserfreiem Äther verrieben, abfiltriert und getrocknet. So wurde 0,48 g (82% der Theorie) des freien Hydroxyacetylheptapeptidmethylesters
Hydroxyacetyl-L-arginyl-L-valyl-L-tyrosyl-L-isoleucyl-L-histidyl-L-prolyl-L-isoleucinmethylester
erhalten. Dieses rohe Produkt wurde dann nach der vor den Beispielen beschriebenen allgemeinen Verfahrensweise gereinigt.

Das erhaltene reine Hydroxyacetyl-L-arginyl-L-valyl-L-tyrosyl-L-isoleucyl-L-histidyl-L-prolyl-L-isoleucinmethylester
hatte die folgenden charakteristischen Eigenschaften: $R_f^7$-Wert = 0,29.
$R_f^8$-Wert = 0,72.
$R_f^9$-Wert = 0,60.

Aminosäureanalyse:

| | | |
|---|---|---|
| L – Prolin | = | 1,0 [1], |
| L – Valin | = | 1,0 [1], |
| L – Isoleucin | = | 1,7 [2], |
| L – Tyrosin | = | 0,88 [1], |
| L – Histidin | = | 1,02 [1], und |
| L – Arginin | = | 0,96 [1]. |

Beispiel 3
L-α-Aminooxypropionyl-L-arginyl-L-valyl-L-tyrosyl-L-isoleucyl-L-histidyl-L-prolyl-L-isoleucinmethylester
{[L-α-Aminooxypropionsäure[1], Isoleucin-methylester[8]]-angiotensin-II}

Schritt 1:
Boc-Arg/Tos/-Val-Tyr/Bzl/-Ile-His/Dnp/-Pro-OBzl

2,9 g /12 mMol/ Pro-OBzl.HCl werden in 50 ml Chloroform gelöst und es werden 1,68 ml Triäthylamin und 5,87 g /10 mMol/ Boc-His/Dnp/-OPfp zugesetzt. Die Lösung wird bei konstant gehaltenem pH-Wert eine Stunde gerührt, dann mit je 25 ml 10%iger wässriger Citronensäurelösung, n Salzsäurelösung, 5%iger wässriger Natriumhydrogencarbonatlösung und Wasser in der angegebenen Reihenfolge ausgeschüttelt, mit wasserfreiem Natriumsulfat getrocknet und eingedampft. Der Rückstand wird mit n-Hexan verrieben und abfiltriert. Das erhaltene geschützte Dipeptid /$R_f$/3/ = 0,80/ wird in 13 ml Salzsäurelösung in Dioxan gelöst und nach 15 Minuten Stehen wird das Produkt durch die Zugabe von wasserfreiem Äther gefällt, abfiltriert und mit Äther gewaschen. Das auf diese Weise erhaltene freie Dipeptid-hydrochlorid /$R_f$/4/ = 0,24/ wird in 30 ml Dimethylformamid sofort gelöst, der pH-Wert der Lösung wird mit Triäthylamin auf 8 eingestellt und es werden 4,7 g /12 mMol/ Boc-Ile-OPfp zugesetzt. Die Lösung wird bei konstant gehaltenem pH-Wert eine Stunde stehen gelassen, dann wird das Lösungsmittel verdampft, der Rückstand wird in Chloroform gelöst, die Lösung wird mit 10%iger wässriger Citronensäurelösung, n Salzsäurelösung, 5%iger wässriger Natriumhydrogencarbonatlösung und mit Wasser in der angegebenen Reihenfolge ausgeschüttelt, mit wasserfreiem Natriumsulfat getrocknet und eingedampft. Der Rückstand wird mit einem 1 : 2 Gemisch von Äther und n-Hexan verrieben und abfiltriert. Das erhaltene geschützte Tripeptid /$R_f$/3/ = 0,82/ wird in 15 ml 8n Salzsäurelösung in Dioxan gelöst und nach 15 Minuten Stehen wird das Produkt durch die Zugabe von wasserfreiem Äther gefällt, abfiltriert und mit Äther gewaschen. Das erhaltene freie Tripeptidhydrochlorid /$R_f$/5/ = 0,38/ wird in 50 ml Dimethylformamid sofort gelöst, der pH-Wert der Lösung wird mit Triäthylamin auf 8 eingestellt und es werden 5,9 g /11 mMol/ Boc-Tyr/Bzl/-OPfp zugesetzt. Die Lösung wird bei konstant gehaltenem pH-Wert 30 Minuten stehen gelassen, dann wird das Lösungsmittel verdampft, der Rückstand wird in Äthylacetat gelöst, mit 0,22 ml N,N-Dimethylamino-äthylamin versetzt und 15 Minuten stehen gelassen. Das Reaktionsgemisch wird dann mit 10%iger wässriger Citronensäurelösung, mit n Salzsäurelösung und zuletzt mit Wasser ausgeschüttelt, mit wasserfreiem Natriumsulfat getrocknet und eingedampft. Der Rückstand wird mit Äther verrieben und abfiltriert. Das auf diese Weise erhaltene geschützte Tetrapeptid /$R_f$/3/ = 0,73/ wird in 20 ml Salzsäurelösung in Dioxan sofort gelöst und nach 15 Minuten Stehen wird das Produkt durch die Zugabe von wasserfreiem Äther gefällt, abfiltriert und gewaschen. Das erhaltene freie Tetrapeptid-hydrochlorid ($R_f^{(5)}$ = 0,69) wird in 50 ml Dimethylformamid sofort gelöst, der pH-Wert der Lösung wird mit Triäthylamin auf 8 eingestellt und es werden 4,2 g (11 mMol) Boc-Val-OPfp zugesetzt. Die Lösung wird bei konstant gehaltenem pH-Wert eine Stunde stehen gelassen, dann wird das Lösungsmittel verdampft, der Rückstand wird in Äthylacetat gelöst, die Lösung wird in der üblichen Weise ausgeschüttelt, getrocknet und eingedampft. Der Rückstand wird mit n-Hexan und anschliessend mit wasserfreiem Äther behandelt und abfiltriert. Das erhaltene geschützte Pentapeptid ($R_f^{(3)}$ = 0,65) wird in 20 ml 8n Salzsäurelösung in Dioxan gelöst, die Lösung wird 15 Minuten stehen gelassen, dann mit wasserfreiem Äther versetzt und das gefällte Produkt wird abfiltriert und gewaschen. Das auf diese Weise erhaltene freie Pentapeptid-hydrochlorid ($R_f^{(5)}$ = 0,57) wird in 60 ml Dimethylformamid sofort gelöst, der pH-Wert der Lösung wird mit Triäthylamin auf 8 eingestellt und es werden 5,94 g (10 mMol) Boc-Arg-(Tos)-OPfp zugesetzt. Die Lösung wird bei konstant gehaltenem pH-Wert eine Stunde stehen gelassen, dann wird das Lösungsmittel verdampft, der Rückstand wird in Chloroform gelöst, die Lösung mit 10%iger wässriger Citronensäurelösung, mit n Salzsäurelösung, mit 5%iger wässriger Natriumhydrogencarbonatlösung und mit Wasser in der angegebenen Reihenfolge ausgeschüttelt, mit wasserfreiem Natriumsulfat getrocknet und eingedampft. Der Rückstand wird mit wasserfreiem Äther verrieben und abfiltriert. Es werden auf diese Weise 5,8 g Boc-Arg(Tos)-Val-Tyr-(Bzl)-Ile-His(Dnp)-Pro-OBzl ($R_f^{(3)}$ = 0,63) erhalten (42% der auf Bis berechneten theoretischen Ausbeute, was einer schrittweisen Durchschnittsausbeute von 84% entspricht); F. 167–174 °C.

Schritt 2:
Boc-Arg(Tos)-Val-Tyr(Bzl)-Ile-His-Pro-OH

2,8 g (2,1 mMol) des auf obige Weise erhaltenen geschützten Hexapeptids Boc-Arg(Tos)-Val-Tyr-(Bzl)-Ile-His(Dnp)-Pro-OBzl werden in 8 ml Dimethylformamid gelöst, mit 2,9 ml 2-Mercaptoäthanol versetzt und eine Stunde gerührt. Dann wird das von der Dinitrophenyl-Schutzgruppe befreite Produkt ($R_f^{(4)}$ = 0,17) durch die Zugabe von wasserfreiem Äther gefällt, in 30 ml Dioxan suspendiert und mit 12 ml n Natriumhydroxydlösung versetzt. Das Gemisch wird eine Stunde stehen gelassen, dann wird der pH-Wert der erhaltenen klaren Lösung mit n Salzsäure auf 7 eingestellt und das Dioxan wird abdestilliert. Der pH-Wert der verbliebenen wässrigen Lösung wird mit Salzsäure auf 3 eingestellt, der erhaltene Niederschlag wird abgetrennt und in 60 ml eines 2 : 3 Gemisches von Dimethylformamid und Chloroform gelöst. Die organische Phase der erhaltenen Lösung wird abgetrennt und die organischen Lösungsmittel werden abdestilliert. Es werden als Rückstand 2,3 g Boc-Arg/Tos/-Val-Tyr/Bzl/-Ile-His-Pro-OH /96% d. Th./ erhalten: $R_f$/5/ = 0,37; F. 160–164 °C Zers./.

Schritt 3:
Boc-Arg/Tos/-Val-Tyr/Bzl/-Ile-His-Pro-Ile-OMe

1,25 g /1,1 mMol/ des im Schritt 2 erhaltenen Produkts werden in 15 ml Dimethylformamid gelöst und die Lösung mit 0,5 ml /3,6 mMol/ Triäthylamin, 0,65 g /3,6 mMol/ Ile-OMe-hydrochlorid und 1,35 g /2 mMol/ des kristallinen Komplexes von Dicyclohexylcarbodiimid und Pentafluorphenol /Molverhältnis der Komponenten 1 : 3/ versetzt. Das Gemisch wird bei konstant gehaltenem pH-Wert stehen gelassen und nach 24 Stunden mit

einer weiteren gleichen Menge des obigen Komplexes und mit weiteren 0,65 g /3,6 mMol/ Ile-OMe-hydrochlorid versetzt. Nach weiteren 24 Stunden wird das Reaktionsgemisch mit 45 ml Chloroform verdünnt und mit je 20 ml 10%iger wässriger Citronensäurelösung zweimal, dann mit 5%iger wässriger Natriumhydrogencarbonatlösung und zuletzt mit Wasser je einmal ausgeschüttelt, mit wasserfreiem Natriumsulfat getrocknet und eingedampft. Der Rückstand wird mit wasserfreiem Äther behandelt und abfiltriert. Der auf diese Weise erhaltene geschützte Heptapeptidester /$R_f$/$^{10}$/ = 0,26/ wird in 20 ml heissem Äthanol gelöst und nach dem Abkühlen wird das Produkt abfiltriert. Es werden 0,95 g Boc-Arg/Tos/-Val-Tyr/Bzl/-Ile-His-Pro-Ile-OMe /65% d. Th./ erhalten.

Schritt 4:
Boc-L-Ala-Arg/Tos/-Val-Tyr/Bzl/-Ile-His-Pro-Ile-OMe

0,95 g Boc-Arg/Tos/-Val-Tyr/Bzl/-Ile-His-Pro-Ile-OMe werden in 3 ml 8n Salzsäurelösung in Dioxan gelöst, die Lösung wird 15 Minuten stehen gelassen und dann wird durch die Zugabe von wasserfreiem Äther das freie Heptapeptidester-hydrochlorid /$R_f$/$^{5}$/ = 0,24/ gefällt, mit Äther gewaschen und sofort in 10 ml Dimethylformamid gelöst. Der pH-Wert der Lösung wird mit Triäthylamin auf 8 eingestellt und es werden 0,5 g /1,1 mMol/ Boc-OAla-OPfp zugesetzt. Die Lösung wird bei konstant gehaltenem pH-Wert eine Stunde stehen gelassen, dann mit 30 ml Chloroform verdünnt, mit 10%iger wässriger Citronensäurelösung und mit Wasser ausgeschüttelt, mit wasserfreiem Natriumsulfat getrocknet und eingedampft. Der erhaltene geschützte α-Aminooxypropionyl-heptapeptidester wird mit Äther verrieben und abfiltriert. Es werden auf diese Weise 0,83 g
Boc-L-OAla-Arg/Tos/-Val-Tyr/Bzl/-Ile-His-Pro-Ile-OMe
(87% d. Th.) erhalten; $R_f^{(4)}$ = 0,34; F. 143–146 °C (Zers.).

Schritt 5:
Das Entfernen der Schutzgruppen

0,75 g (0,57 mMol) des auf obige Weise erhaltenen geschützten α-Aminooxypropionylheptapeptids werden im Gemisch von 0,5 ml Thioanisol und 2 ml flüssigem Fluorwasserstoff gelöst. Das Gemisch wird 1,5 Stunden bei 0 °C gehalten, dann wird durch die Zugabe von wasserfreiem Äther der freie α-Aminooxypropionylheptapeptidester gefällt. Dieses Produkt wird in 30 ml Methanol gelöst und durch die Zugabe von wasserfreiem Äther wieder gefällt. Es werden auf diese Weise 0,5 g roher α-Aminooxypropionylheptapeptidester (89% d. Th.) erhalten. Dieses rohe Produkt wird dann nach der oben beschriebenen allgemeinen Reinigungsmethode gereinigt. Das erhaltene reine (L-α-Aminooxypropionsäure$^1$, Isoleucin-methylester$^8$)-Angiotensin-II zeigt die folgenden charakteristischen Eigenschaften:

$R_f^{(7)}$ = 0,30; $R_f^{(8)}$ = 0,68; $R_f^{(9)}$ = 0,46. Aminosäure-Analyse: Pro 1,0 (1), Val 1,05 (1), Ile 2,0 (2), Tyr 0,73 (1), His 0,85 (1), Arg 0,97 (1).

Beispiel 4
L-Sarkosyl-L-arginyl-L-valyl-L-tyrosyl-L-isoleucyl-L-histidyl-L-prolyl-L-methylthreoninmethylester
{(Sarcosin$^1$, Threonin[Me]-methylester$^8$)-angiotensin-II}

1. Stufe
[N-(Benzyloxycarbonyl)]-L-sarkosyl-[N-(nitro)]-L-arginyl-L-valyl-[O-(benzyl)]-L-tyrosyl-L-isoleucyl-[N-(dinitrophenyl)]-L-histidyl-L-prolyl-L-methylthreoninmethylester

Es wurde 0,68 g (4,5 Millimol) L-Methylthreoninmethylester in 10 $cm^3$ Chloroform gelöst und mit 2,28 g (6 Millimol)
[N-(tert.-Butyloxycarbonyl)]-L-prolinpentafluorphenylester
versetzt. Die Lösung wurde bei einem konstant gehaltenen pH-Wert von 8 ½ Stunde stehengelassen, dann wurde das Lösungsmittel abdestilliert und der Rückstand in Äthylacetat gelöst und es wurde 0,22 $cm^3$ N,N-Dimethylaminoäthylamin zugesetzt. Nach 15 Minuten langem Stehen wurde das Reaktionsgemisch zuerst mit einer mit Natriumchlorid gesättigten 10%igen Citronensäurelösung, dann mit einer n Salzsäure und schliesslich mit Wasser ausgeschüttelt, über wasserfreiem Natriumsulfat getrocknet und eingedampft. Der als Rückstand verbliebene geschützte Dipeptidester
[N-(tert.-Butyloxycarbonyl)]-L-prolyl-L-methylthreoninmethylester ($R_f^2$-Wert = 0,76) wurde ohne Reinigung in 3 $cm^3$ einer 8n Lösung von Chlorwasserstoff in Dioxan gelöst und die Lösung wurde 15 Minuten stehengelassen, dann mit wasserfreiem Äther verdünnt und eingedampft. Das als Rückstand erhaltene freie Dipeptidesterhydrochlorid L-Prolyl-L-methylthreoninmethylester-hydrochlorid ($R_f^4$-Wert = 0,18) wurde sofort in 10 $cm^3$ Dimethylformamid gelöst, der pH-Wert der Lösung wurde mit Triäthylamin auf 8 eingestellt und es wurden 2,95 g (5 Millimol)
[N-(tert.-Butyloxycarbonyl)-N′-(dinitrophenyl)]-L-histidinpentafluorphenylester
zugesetzt. Die Lösung wurde bei einem konstant gehaltenen pH-Wert von 8 ½ Stunde stehengelassen, dann wurde das Lösungsmittel abdestilliert und der Rückstand wurde in Äthylacetat gelöst. Die Lösung wurde mit 0,11 $cm^3$ N,N-Dimethylaminoäthylamin versetzt, 10 Minuten stehengelassen, dann mit einer mit Natriumchlorid gesättigten 10%igen wässrigen Citronensäurelösung und anschliessend mit Wasser ausgeschüttelt, über wasserfreiem Natriumsulfat getrocknet und eingedampft. Der als Rückstand verbliebene geschützte Tripeptidester
[N-(tert.-Butyloxycarbonyl)-N′-(dinitrophenyl)]-L-histidyl-L-prolyl-L-methylthreoninmethylester
($R_f^2$-Wert = 0,47) wurde sofort in 5 $cm^3$ einer 8n Lösung von Chlorwasserstoff in Dioxan gelöst und nach 15 Minuten langem Stehenlassen wurde das Reaktionsprodukt durch Zugabe von wasserfreiem Äther gefällt, abfiltriert und mit Äther gewaschen. Das so erhaltene freie Tripeptidesterhydrochlorid

[N-(Dinitrophenyl)]-L-histidyl-L-prolyl-L-methylthreoninmethylesterhydrochlorid

($R_f^4$-Wert = 0,3) wurde sofort in 10 $cm^3$ Dimethylformamid gelöst, der pH-Wert der Lösung wurde mit Triäthylamin auf 8 eingestellt und es wurden 2,0 g (5 Millimol)

[N-(tert.-Butyloxycarbonyl)]-L-isoleucinpentafluorphenylester

zugesetzt. Die Lösung wurde bei einem konstant gehaltenen pH-Wert von 8 ½ Stunde stehengelassen und dann wurde das Lösungsmittel abdestilliert, der Rückstand in Äthylacetat gelöst und die Lösung in der üblichen Weise ausgeschüttelt, getrocknet und eingedampft. Der Rückstand wurde mit einem Gemisch von Äther und n-Hexan im Volumverhältnis von 3 : 7 verrieben und abfiltriert. Der so erhaltene geschützte Tetrapeptidester

[N-(tert.-Butyloxycarbonyl)]-L-isoleucyl-[N-(dinitrophenyl)]-L-histidyl-L-prolyl-L-methylthreoninmethylester

($R_f^2$-Wert = 0,28) wurde sofort in 7 $cm^3$ einer 8n Lösung von Chlorwasserstoff in Dioxan gelöst, die Lösung wurde 15 Minuten stehengelassen und dann wurde durch Zugabe von wasserfreiem Äther das freie Tetrapeptidesterhydrochlorid L-Isoleucyl-[N-(dinitrophenyl)]-L-histidyl-L-prolyl-L-methylthreoninmethylesterhydrochlorid

gefällt, abfiltriert und mit Äther gewaschen. Dieses Produkt ($R_f^5$-Wert = 0,27) wurde in 10 $cm^3$ Dimethylformamid gelöst, der pH-Wert der Lösung wurde mit Triäthylamin auf 8 eingestellt und es wurden 2,7 g (5 Millimol)

[N-(tert.-Butyloxycarbonyl)-O-(benzyl)]-L-tyrosinpentafluorphenylester

zugesetzt. Die Lösung wurde bei einem konstant gehaltenen pH-Wert von 8 ½ Stunde stehengelassen, dann wurde das Lösungsmittel abdestilliert, der Rückstand wurde in Äthylacetat gelöst und die Lösung wurde mit 0,11 $cm^3$ N,N-Dimethylaminoäthylamin versetzt und nach 15 Minuten langem Stehenlassen mit einer mit Natriumchlorid gesättigten 10%igen wässrigen Citronensäurelösung und mit Wasser ausgeschüttelt, über wasserfreiem Natriumsulfat getrocknet und eingedampft. Der als Rückstand verbliebene geschützte Pentapeptidester

[N-(tert.-Butyloxycarbonyl)-O-(benzyl)]-L-tyrosyl-L-isoleucyl-[N-(dinitrophenyl)]-L-histidyl-L-prolyl-L-methylthreoninmethylester

($R_f^2$-Wert = 0,51) wurde sofort mit wasserfreiem Äther verrieben, abfiltriert und in 5 $cm^3$ einer 8n Lösung von Chlorwasserstoff in Dioxan gelöst. Nach 15 Minuten langem Stehenlassen wurde durch Zugabe von wasserfreiem Äther das freie Pentapeptidesterhydrochlorid [O-(Benzyl)]-L-tyrosyl-L-isoleucyl-[N-(dinitrophenyl)]-L-histidyl-L-prolyl-L-methylthreoninmethylesterhydrochlorid

($R_f^5$-Wert = 0,48) gefällt, abfiltriert, mit Äther gewaschen und in 20 $cm^3$ Dimethylformamid gelöst. Der pH-Wert der Lösung wurde mit Triäthylamin auf 8 eingestellt und es wurden 1,9 g (9 Millimol)

[N-(tert.-Butyloxycarbonyl)]-L-valinpentafluorphenylester

zugesetzt. Die Lösung wurde bei einem konstant gehaltenen pH-Wert von 8 ½ Stunde stehengelassen, dann wurde das Lösungsmittel abdestilliert, der Rückstand wurde in Chloroform gelöst und die Lösung wurde in der üblichen Weise ausgeschüttelt, getrocknet und eingedampft. Der Rückstand wurde mit wasserfreiem Äther verrieben und abfiltriert. Der erhaltene geschützte Hexapeptidester

[N-(tert.-Butyloxycarbonyl]-L-valyl-[O-(benzyl)]-L-tyrosyl-L-isoleucyl-[N-(dinitrophenyl)]-L-histidyl-L-prolyl-L-methyltreoninmethylester

($R_f^2$-Wert = 0,44) wurde in 10 $cm^3$ einer 8n Lösung von Chlorwasserstoff in Dioxan gelöst und nach 15 Minuten langem Stehenlassen wurde das Produkt durch Zugabe von wasserfreiem Äther gefällt, abfiltriert und gewaschen. Das erhaltene freie Hexapeptidesterhydrochlorid

L-Valyl-[O-(benzyl)]-L-tyrosyl-L-isoleucyl-[N-(dinitrophenyl)]-L-histidyl-L-prolyl-L-methylthreoninmethylesterhydrochlorid

($R_f^4$-Wert = 0,36) wurde sofort in 15 $cm^3$ Dimethylformamid gelöst, der pH-Wert der Lösung wurde mit Triäthylamin auf 8 eingestellt und es wurden 2,25 g (5 Millimol)

[N-(tert.-Butyloxycarbonyl)-N′-(nitro)]-L-argininpentafluorphenylester

zugesetzt.

Die Lösung wurde bei einem konstant gehaltenen pH-Wert von 8 1 Stunde stehengelassen, dann mit 45 $cm^3$ Chloroform verdünnt und in der üblichen Weise ausgeschüttelt, getrocknet und eingedampft. Der Rückstand wurde mit Äthanol verrieben und abfiltriert. Es wurden 3,3 g des geschützten Heptapeptidesters

[N-(tert.-Butyloxycarbonyl)-N′-(nitro)]-L-arginyl-L-valyl-[O-(benzyl)]-L-tyrosyl-L-isoleucyl-[N-(dinitrophenyl)]-L-histidyl-L-prolyl-L-methylthreoninmethylester

(Schmelzpunkt = 188 bis 193 °C, $R_f^3$-Wert = 0,38 und $R_f^4$-Wert = 0,87) erhalten. (Dies stellt eine Ausbeute von 56% der Theorie, bezogen auf Threonin, dar, was einer durchschnittlichen Ausbeute von 91% der Theorie je Schritt entspricht.)

Es wurden 1,95 g (1,5 Millimol) des wie vorstehend beschrieben erhaltenen geschützten Heptapeptidesters

[N-(tert.-Butyloxycarbonyl)-N′-(nitro)]-L-arginyl-L-valyl-[O-(benzyl)]-L-tyrosyl-L-isoleucyl-[N-(dinitrophenyl)]-L-histidyl-L-prolyl-L-methylthreoninmethylester

in 10 $cm^3$ einer 8n Lösung von Chlorwasserstoff in Dioxan gelöst und nach 20 Minuten langem Stehenlassen wurde das Produkt durch Zugabe von wasserfreiem Äther gefällt, abfiltriert und gewaschen. Das erhaltene freie Heptapeptidesterhydrochlorid

[N-(Nitro)]-L-arginyl-L-valyl-[O-(benzyl)]-L-tyrosyl-L-isoleucyl-[N-(dinitrophenyl)]-L-histidyl-L-prolyl-L-methyltreoninmethylesterhydrochlorid

($R_f^4$-Wert = 0,20) wurde in 15 $cm^3$ Dimethylformamid gelöst, der pH-Wert der Lösung wurde mit

Triäthylamin auf 8 eingestellt und es wurde 0,87 g (2,25 Millimol)
N-(Benzyloxycarbonyl)-L-sarkosinpentafluorphenylester
zugesetzt. Die Lösung wurde bei einem konstant gehaltenen pH-Wert von 8 1 Stunde stehengelassen, dann mit 45 $cm^3$ Chloroform verdünnt, mit einer 10%igen wässrigen Citronensäurelösung, dann mit einer n Salzsäure und schliesslich mit Wasser ausgeschüttelt, über wasserfreiem Natriumsulfat getrocknet und eingedampft. Der Rückstand wurde mit Äthanol verrieben, abfiltriert und mit Äthanol gewaschen. So wurden 1,85 g (87% der Theorie) des geschützten Octapeptidesters
[N-(Benzyloxycarbonyl)]-L-sarkosyl-[N-(nitro)]-L-arginyl-L-valyl-[O-(benzyl)]-L-tyrosyl-L-isoleucyl-[N-(dinitrophenyl)]-L-histidyl-L-prolyl-L-methylthreoninmethylester
mit einem Schmelzpunkt von 202 bis 208 °C, einem $R_f^3$-Wert von 0,20 und einem $R_f^4$-Wert von 0,86 erhalten.

2. Stufe
L-Sarkosyl-L-arginyl-L-valyl-L-tyrosyl-L-isoleucyl-L-histidyl-L-prolyl-L-methylthreoninmethylester
{Entfernen der Schutzgruppen}

Es wurden 1,85 g (1,4 Millimol) wie in der vorstehenden 1. Stufe beschrieben erhaltener geschützter Octapeptidester
[N-(Benzyloxycarbonyl)]-L-sarkosyl-[N-(nitro)]-L-arginyl-L-valyl-[O-(benzyl)]-L-tyrosyl-L-isoleucyl-[N-(dinitrophenyl)]-L-histidyl-L-prolyl-L-methylthreoninmethylester
in 8 $cm^3$ Dimethylformamid gelöst und mit 2,6 $cm^3$ 2-Mercaptoäthanol versetzt. Das Gemisch wurde 1 Stunde gerührt beziehungsweise geschüttelt und dann wurden durch Zugabe von wasserfreiem Äther 1,6 g des von der Dinitrophenylgruppe befreiten geschützten Octapeptidesters
[N-(Benzyloxycarbonyl)]-L-sarkosyl-[N-(nitro)]-L-arginyl-L-valyl-[O-(benzyl)]-L-tyrosyl-L-isoleucyl-L-histidyl-L-prolyl-L-methylthreoninmethylester

($R_f^4$-Wert = 0,52) gefällt, abfiltriert und gewaschen. Dieses Produkt wurde in 30 $cm^3$ eines Gemisches von Methanol, Essigsäure und Wasser im Volumverhältnis von 5 : 1 : 1 gelöst und es wurde 1,0 g von 10% Palladium auf Aktivkohle als Katalysator zugesetzt. Dann wurde 30 Stunden lang unter kräftigem Rühren beziehungsweise Schütteln Wasserstoffgas durch die Lösung geleitet, worauf der Katalysator abfiltriert und mit demselben Lösungsmittelgemisch gewaschen und das mit der Waschflüssigkeit vereinigte Filtrat zur Trockne eingedampft wurde. Der Rückstand wurde mit einem Gemisch von Äther und Äthanol im Volumverhältnis von 2 : 1 verrieben und abfiltriert. So wurde 0,98 g (83% der Theorie) des freien Octapeptidesters
L-Sarkosyl-L-arginyl-L-valyl-L-tyrosyl-L-isoleucyl-L-histidyl-L-prolyl-L-methylthreoninmethylester
erhalten. Dieses rohe Produkt wurde dann nach der vor den Beispielen beschriebenen allgemeinen Reinigungsverfahrensweise gereinigt. Der erhaltene reine
L-Sarkosyl-L-arginyl-L-valyl-L-tyrosyl-L-isoleucyl-L-histidyl-L-prolyl-L-methylthreoninmethylester
hatte die folgenden charakteristischen Eigenschaften:

$R_f^6$-Wert = 0,29.
$R_f^8$-Wert = 0,55.
$R_f^9$-Wert = 0,27.

Aminosäureanalyse:

| | | | |
|---|---|---|---|
| L – Histidin | = | 1,03 [1], | |
| L – Arginin | = | 0,95 [1], | |
| L – Threonin | = | 0,93 [1], | |
| L – Prolin | = | 1,03 [1], | |
| L – Valin | = | 1,15 [1], | |
| L – Isoleucin | = | 1,03 [1], | |
| L – Tyrosin | = | 0,90 [1] | und |
| L – Sarkosin | = | 1,0 [1]. | |

Beispiel 5
Hydroxyacetyl-L-arginyl-L-valyl-L-tyrosyl-L-isoleucyl-L-histidyl-L-prolyl-L-methylthreoninmethylester
{(Hydroxyessigsäure[1],Threonin(Me)-methylester[8])-Angiotensin-II}

Schritt 1:
Z-OGly-Arg($NO_2$)-Val-Tyr(Bzl)-Ile-His(Dnp)-Pro-Thr(Me)-OMe

0,62 g (0,47 mMol) Boc-Arg($NO_2$)-Val-Tyr(Bzl)-Ile-His(Dnp)-Pro-Thr(Me)-OMe (hergestellt nach Beispiel 4, Schritt 1) werden in 4 ml 8n Salzsäurelösung in Dioxan gelöst und nach 15 Minuten Stehen wird durch die Zugabe von wasserfreiem Äther das freie Heptapeptidester-hydrochlorid gefällt, abfiltriert und mit Äther gewaschen. Dieses Produkt ($R_f^{(5)}$ = 0,35) wird in 10 ml Dimethylformamid sofort gelöst, der pH-Wert der Lösung wird mit Triäthylamin auf 8 eingestellt und es werden 0,8 g (2 mMol) Z-OGly-OPfp zugesetzt. Die Lösung wird bei dem konstant gehaltenen pH-Wert 8 eine Stunde stehen gelassen, dann wird sie mit 30 ml Chloroform verdünnt, mit 10%iger wässriger Citronensäurelösung, dann mit n Salzsäurelösung und zuletzt mit Wasser ausgeschüttelt, mit wasserfreiem Natriumsulfat getrocknet und eingedampft. Der Rückstand wird mit einem 9 : 1 Gemisch von Äther und Äthanol verrieben und abfiltriert. Es werden auf diese Weise 0,60 g geschützter Aminooxyacetylheptapeptidester
Z-OGly-Arg/$NO_2$/-Val-Tyr/Bzl/-Ile-His/Dnp/-Pro-Thr/Me/-OMe
/90% d. Th./ erhalten; F. 158–162 °C; $R_f^{/3/}$ = 0,44.

Schritt 2:
Das Entfernen der Schutzgruppen

0,6 g /0,42 mMol/ geschützter Aminooxyacetylheptapeptidester werden in 2 ml Dimethylformamid gelöst, mit 1,2 ml 2-Mercaptoäthanol versetzt und nach einer Stunde Stehen wird der von der Dinitrophenylgruppe befreite geschützte Aminooxyacetylheptapeptidester durch die Zugabe von

wasserfreiem Äther gefällt. Dieses Produkt wird in Methanol gelöst, die Lösung wird mit Aktivkohle geklärt, filtriert und zur Trockne eingedampft. Der Rückstand wird mit Äther verrieben und abfiltriert. Ausbeute: 0,46 g; $R_f$/4/ = 0,16; $R_f$/5/ = 0,52/.

Das Produkt wird in einem 5 : 1 : 1 Gemisch von Methanol, Essigsäure und Wasser /25 ml/ gelöst, mit 0,3 g 10%igem Palladium-Aktivkohle-Katalysator versetzt und Wasserstoffgas wird unter lebhaftem Rühren 17 Stunden lang durch das Gemisch geleitet. Nach der Beendigung der Reaktion wird der Katalysator abfiltriert, mit dem selben Lösungsmittelgemisch nachgewaschen und das mit der Waschflüssigkeit vereinigte Filtrat wird zur Trockne eingedampft. Der Rückstand wird mit einem Gemisch von Wasser und Äthanol mehrmals versetzt und zur Trockne eingedampft, dann mit Äther verrieben und abfiltriert. Es werden 0,32 g freier Hydroxyacetylheptapeptidester /91% d. Th./ erhalten. Dieses rohe Produkt wird nach der oben beschriebenen allgemeinen Reinigungsmethode gereinigt; das auf diese Weise erhaltene reine /Hydroxyessigsäure¹,Threonin/Me/-methylester⁸/Angiotensin-II zeigt die folgenden charakteristischen Eigenschaften: $R_f$/7/ = 0,22; $R_f$/8/ = 0,73; $R_f$/9/ = 0,56.

Aminosäure-Analyse: Thr 0,92 /1/, Pro 1,03 /1/, Val 1,0 /1/, Tyr 0,7 /1/, Ile 1,05 /1/, His 1,0 /1/, Arg 1,0 /1/.

Beispiel 6

Hydroxyacetyl-L-arginyl-L-valyl-L-tyrosyl-L-isoleucyl-L-histidyl-L-prolyl-L-threoninmethylester

{Hydroxyessigsäure¹,Threonin-methylester⁸/-Angiotensin-II}

Schritt 1:

Boc-Arg/$NO_2$/-Val-Tyr/Bzl/-Ile-His/Dnp/-Pro-Thr-OMe

3,8 g /20 mMol/ Thr-OMe.HCl werden in 50 ml Chloroform gelöst und es werden 2,8 ml /20 mMol/ Triäthylamin und 3,8 g /10 mMol/ Boc-Pro-OPfp zugesetzt. Die Lösung wird bei konstant gehaltenem pH-Wert eine Stunde stehen gelassen, dann mit 10%iger wässriger Citronensäurelösung, dann mit n Salzsäure und zuletzt mit Wasser ausgeschüttelt, mit wasserfreiem Natriumsulfat getrocknet und eingedampft. Der erhaltene geschützte Dipeptidester ($R_f^{(3)}$ = 0,77) wird in 20 ml 8n Salzsäurelösung in Dioxan gelöst und nach 15 Minuten Stehen wird durch die Zugabe von wasserfreiem Äther das freie Dipeptidester-hydrochlorid gefällt, abfiltriert und gewaschen. Dieses Produkt ($R_f^{(5)}$ = 0,2) wird in 30 ml Chloroform sofort gelöst, der pH-Wert der Lösung wird mit Triäthylamin auf 8 eingestellt und es werden 4,7 g (8 mMol) Boc-His(Dnp)-OPfp zugesetzt. Die Lösung wird bei dem konstant gehaltenen pH-Wert 8 eine Stunde stehen gelassen, dann mit 10%iger wässriger Citronensäurelösung, mit n Salzsäurelösung, mit 5%iger wässriger Natriumhydrogencarbonatlösung und mit Wasser in der angegebenen Reihenfolge ausgeschüttelt, mit wasserfreiem Natriumsulfat getrocknet und eingedampft. Der als Rückstand erhaltene geschützte Tripeptidester ($R_f^{(3)}$ = 0,47) wird in 20 ml 8n Salzsäurelösung in Dioxan gelöst und nach 15 Minuten Stehen wird durch die Zugabe von wasserfreiem Äther das freie Tripeptidester-hydrochlorid ($R_f^{(5)}$ = 0,23) gefällt. Dieses Produkt wird in 20 ml eines 5 : 1 Gemisches von Chloroform und Dimethylformamid gelöst, der pH-Wert der Lösung wird mit Triäthylamin auf 8 eingestellt und es werden 4,0 g (10 mMol) Boc-Ile-OPfp zugesetzt. Die Lösung wird bei dem konstant gehaltenen pH-Wert 8 eine halbe Stunde stehen gelassen, dann mit 10%iger wässriger Citronensäurelösung, dann mit 5%iger wässriger Natriumhydrogencarbonatlösung und schliesslich mit Wasser ausgeschüttelt, mit wasserfreiem Natriumsulfat getrocknet und eingedampft. Der Rückstand wird mit n-Hexan mehrmals verrieben und abfiltriert. Der auf diese Weise erhaltene geschützte Tetrapeptidester ($R_f^{(3)}$ = 0,45) wird in 15 ml 8n Salzsäurelösung in Dioxan gelöst und nach 15 Minuten Stehen wird durch die Zugabe von wasserfreiem Äther das freie Tetrapeptidesterhydrochlorid gefällt. Dieses Produkt ($R_f^{(5)}$ = 0,25) wird in 30 ml Dimethylformamid sofort gelöst, der pH-Wert der Lösung wird mit Triäthylamin auf 8 eingestellt und es werden 2,05 g (3,8 mMol) Boc-Tyr(Bzl)-OPfp zugesetzt. Die Lösung wird bei dem konstant gehaltenen pH-Wert 8 eine halbe Stunde stehen gelassen, dann eingedampft, der Rückstand wird in Äthylacetat gelöst und mit 0,11 ml N,N-Dimethylamino-äthylamin versetzt. Nach 15 Minuten Stehen wird die Lösung auf die übliche Weise ausgeschüttelt, getrocknet und eingedampft. Der als Rückstand erhaltene geschützte Pentapeptidester ($R_f^{(3)}$ = 0,57) wird mit wasserfreiem Äther verrieben und abfiltriert, dann in 10 ml 8n Salzsäurelösung in Dioxan gelöst. Nach 15 Minuten Stehen wird durch die Zugabe von wasserfreiem Äther das freie Pentapeptidesterhydrochlorid gefällt, abfiltriert und mit Äther gewaschen. Dieses Produkt ($R_f^{(5)}$ = 0,27) wird in 20 ml Dimethylformamid sofort gelöst, der pH-Wert der Lösung wird mit Triäthylamin auf 8 eingestellt und es werden 1,75 g (4,5 mMol) Boc-Val-OPfp zugesetzt. Die Lösung wird bei dem konstant gehaltenen pH-Wert 8 eine Stunde stehen gelassen, dann wird das Lösungsmittel verdampft, der Rückstand wird in Chloroform gelöst und die Lösung wird auf die übliche Weise ausgeschüttelt, getrocknet und eingedampft. Der ölige Rückstand wird mit Äther verrieben, abfiltriert und gewaschen. Der auf diese Weise erhaltene geschützte Hexapeptidester ($R_f^{(3)}$ = 0,55) wird in 8 ml 8n Salzsäurelösung in Dioxan gelöst und nach 15 Minuten Stehen wird das freie Hexapeptidesterhydrochlorid durch die Zugabe von wasserfreiem Äther gefällt, abfiltriert und mit Äther gewaschen. Dieses Produkt ($R_f^{(5)}$ = 0,23) wird in 20 ml Dimethylformamid sofort gelöst, der pH-Wert der Lösung wird mit Triäthylamin auf 8 eingestellt und es werden 1,8 g (4 mMol) Boc-Arg($NO_2$)-OPfp zugesetzt. Die Lösung wird bei dem konstant gehaltenen pH-Wert 8 eine Stunde stehen gelassen, dann wird das Lösungsmittel verdampft, der Rückstand wird in Chloroform gelöst und die Lösung wird auf

die übliche Weise ausgeschüttelt, getrocknet und eingedampft. Der Rückstand wird in einem 9 : 1 Gemisch von Äther und Äthanol verrieben, abfiltriert und gewaschen. Es werden 2,7 g geschützter Heptapeptidester Boc-Arg/$NO_2$/-Val-Tyr/Bzl/-Ile-His/Dnp/-Pro-Thr-OMe /26% der auf His berechneten theoretischen Ausbeute, was einer durchschnittlichen schrittweisen Ausbeute von 80% entspricht/ erhalten, F. 190–195 °C /Zers./; $R_f^{/4/}$ = 0,47.

Schritt 2:
Z-OGly-Arg/$NO_2$/-Val-Tyr/Bzl/-Ile-His/Dnp/-Pro-Thr-OMe

1,7 g /1,4 mMol/ des obigen geschützten Heptapeptidesters werden in 10 ml 8n Salzsäurelösung in Dioxan gelöst und nach 15 Minuten Stehen wird durch die Zugabe von wasserfreiem Äther das freie Heptapeptidester-hydrochlorid /$R_f^{/5/}$ = 0,21/ gefällt und sofort in 20 ml Dimethylformamid gelöst. Der pH-Wert der Lösung wird mit Triäthylamin auf 8 eingestellt und es werden 0,82 g /2,1 mMol/ Z-OGly-OPfp zugesetzt. Die Lösung wird bei dem konstant gehaltenen pH-Wert 8 eine halbe Stunde stehen gelassen, dann mit 40 ml Chloroform verdünnt, mit n Salzsäurelösung und mit Wasser ausgeschüttelt, getrocknet und eingedampft. Der Rückstand wird mit wasserfreiem Äther verrieben und abfiltriert. Es werden 1,6 g geschützter Aminooxyacetylheptapeptidester /87% d.Th./ erhalten; F. 188–194 °C; $R_f^{/4/}$ = 0,48.

Schritt 3:
Das Entfernen der Schutzgruppen

1,6 g /1,2 mMol/ geschützter Aminooxyacetylheptapeptidester
Z-OGly-Arg/$NO_2$/-Val-Tyr/Bzl/-Ile-His/Dnp/-Pro-Thr-OMe
werden in 5 ml Dimethylformamid gelöst und mit 3,8 ml 2-Mercaptoäthanol versetzt. Das Gemisch wird eine Stunde stehen gelassen, dann wird der von der Dinitrophenylgruppe befreite Aminooxyacetylheptapeptidester durch die Zugabe von wasserfreiem Äther gefällt, abfiltriert, in Methanol gelöst und durch die Zugabe von Äther wieder gefällt. Ausbeute: 1,3 g /94% d.Th./; $R_f^{/4/}$ = 0,35; $R_f^{/5/}$ = 0,72.

Dieses Produkt wird in einem 5 : 1 : 1 Gemisch von Methanol, Essigsäure und Wasser gelöst, es werden 0,6 g 10%iger Palladium-Aktivkohle-Katalysator zugesetzt und Wasserstoffgas wird unter lebhaftem Rühren 17 Stunden lang durch das Gemisch geleitet.

Nach der Beendigung der Reaktion wird der Katalysator abfiltriert und mit dem selben Lösungsmittelgemisch nachgewaschen; das mit der Waschflüssigkeit vereinigte Filtrat wird zur Trockne eingedampft. Der Rückstand wird mit einem Gemisch von Äthanol und Wasser mehrmals eingedampft; der schliesslich erhaltene Rückstand wird mit Äther verrieben, abfiltriert und mit Äther gewaschen. Es werden 0,64 g freier Hydroxyacetylheptapeptidester /92% d.Th./ erhalten. Dieses rohe Produkt wird dann nach der oben beschriebenen allgemeinen Reinigungsmethode gereinigt; der auf diese Weise erhaltene reine Hydroxyacetylheptapeptidester /Hydroxyessigsäure[1], Threonin-methylester[8]/Angiotensin-II zeigt die folgenden charakteristischen Eigenschaften: $R_f^{/7/}$ = 0,22; $R_f^{/8/}$ = 0,73; $R_f^{/9/}$ = 0,56; Aminosäure-Analyse: Thr 0,92 /1/, Pro 1,03 /1/, Val 1,0 /1/, Ile 1,05 /1/, Tyr 0,7 /1/, His 1,0 /1/, Arg 1,0 /1/.

Beispiel 7
L-Sarkosyl-L-arginyl-L-valyl-L-tyrosyl-L-isoleucyl-L-histidyl-L-prolyl-L-alaninmethylester
{/Sarcosin[1],Alanin-methylester[8]/-Angiotensin-II}

1. Stufe
[N-(Benzyloxycarbonyl)]-L-sarkosyl-[N-(nitro)]-L-arginyl-L-valyl-[O-(benzyl)]-L-tyrosyl-L-isoleucyl-[N-(dinitrophenyl)]-L-histidyl-L-prolyl-L-alaninmethylester

Es wurde 0,7 g (5 Millimol) L-Alaninmethylester-hydrochlorid in 20 $cm^3$ Chloroform gelöst, mit 0,7 $cm^3$ Triäthylamin und 1,14 g (3 Millimol)
[N-(tert.-Butyloxycarbonyl)]-L-prolinpentafluorphenylester
versetzt und bei konstant gehaltenem pH-Wert ½ Stunde stehengelassen. Dann wurde die Lösung mit einer 10%igen wässrigen Citronensäurelösung, danach mit einer n Salzsäure und schliesslich mit Wasser ausgeschüttelt, über wasserfreiem Natriumsulfat getrocknet und eingedampft. Der als Rückstand verbliebene geschützte Dipeptidester
[N-(tert.-Butyloxycarbonyl)]-L-prolyl-L-alaninmethylester
($R_f^2$-Wert = 0,61) wurde in 5 $cm^3$ einer 8n Lösung von Chlorwasserstoff in Dioxan gelöst und nach 10 Minuten langem Stehenlassen wurde die Lösung mit wasserfreiem Äther verdünnt und eingedampft. Das als Rückstand verbliebene freie Dipeptidesterhydrochlorid
L-Prolyl-L-alaninmethylesterhydrochlorid
($R_f^5$-Wert = 0,32) wurde in 20 $cm^3$ Chloroform gelöst, der pH-Wert der Lösung wurde mit Triäthylamin auf 8 eingestellt und es wurden 2,7 g (4,5 Millimol)
[N-(tert.-Butyloxycarbonyl)-N′-(dinitrophenyl)]-L-histidinpentafluorphenylester
zugesetzt. Die Lösung wurde bei einem konstant gehaltenen pH-Wert von 8 eine Stunde stehengelassen, dann wurde sie mit 0,33 $cm^3$ Dimethylaminoäthylamin versetzt und nach 15 Minuten langem Stehenlassen wurde das Gemisch mit einer 10%igen wässrigen Citronensäurelösung, mit einer n Salzsäure, mit einer 5%igen wässrigen Natriumbicarbonatlösung und mit Wasser in der angegebenen Reihenfolge ausgeschüttelt, über wasserfreiem Natriumsulfat getrocknet und eingedampft. Der als Rückstand verbliebene geschützte Tripeptidester
[N-(tert.-Butyloxycarbonyl)-N′-(dinitrophenyl)]-L-histidyl-L-prolyl-L-alaninmethylester
($R_f^2$-Wert = 0,27) wurde in 10 $cm^3$ einer 8n Lösung von Chlorwasserstoff in Dioxan gelöst und nach 15

Minuten langem Stehenlassen wurde durch Zugabe von wasserfreiem Äther das freie Tripeptidesterhydrochlorid

[N-(Dinitrophenyl)]-L-histidyl-L-prolyl-L-alaninmethylesterhydrochlorid

($R_f^5$-Wert = 0,48) gefällt, abfiltriert und mit Äther gewaschen. Dieses Produkt wurde sofort in 30 $cm^3$ eines Gemisches von Chloroform und Dimethylformamid im Volumverhältnis von 2 : 1 gelöst, der pH-Wert der Lösung wurde mit Triäthylamin auf 8 eingestellt und es wurden 2,4 g (6 Millimol)

[N-(tert.-Butyloxycarbonyl)]-L-isoleucinpentafluorphenylester

zugesetzt. Die Lösung wurde bei einem konstant gehaltenen pH-Wert von 8 ½ Stunde stehengelassen und dann wurde das Lösungsmittel abgedampft, der Rückstand in Äthylacetat gelöst und die Lösung in der üblichen Weise ausgeschüttelt, getrocknet und eingedampft. Der Rückstand wurde mit einem Gemisch von Äther und n-Hexan im Volumverhältnis von 1 : 9 verrieben und abfiltriert. Der auf diese Weise erhaltene geschützte Tetrapeptidester

[N-(tert.-Butyloxycarbonyl)]-L-isoleucyl-[N-(dinitrophenyl)]-L-histidyl-L-prolyl-L-alaninmethylester

($R_f^2$-Wert = 0,29) wurde in 10 $cm^3$ einer 8n Lösung von Chlorwasserstoff in Dioxan gelöst und nach 10 Minuten langem Stehenlassen wurde durch Zugabe von wasserfreiem Äther das freie Tetrapeptidesterhydrochlorid

L-Isoleucyl-[N-(dinitrophenyl)]-L-histidyl-L-prolyl-L-alaninmethylesterhydrochlorid

($R_f^5$-Wert = 0,67) gefällt, abfiltriert, gewaschen und sofort in einem Gemisch von Chloroform und Dimethylformamid im Volumverhältnis von 2 : 1 gelöst. Der pH-Wert der Lösung wurde mit Triäthylamin auf 8 eingestellt und es wurden 1,78 g (3,3 Millimol)

[N-(tert.-Butyloxycarbonyl)-O-(benzyl)]-L-tyrosinpentafluorphenylester

zugesetzt. Die Lösung wurde bei konstant gehaltenem pH-Wert 15 Minuten stehengelassen, dann wurde das Lösungsmittel abgedampft, der Rückstand in Äthylacetat gelöst und die Lösung in der üblichen Weise ausgeschüttelt, getrocknet und eingedampft. Der Rückstand wurde mit wasserfreiem Äther verrieben und abfiltriert. Der erhaltene geschützte Pentapeptidester

[N-(tert.-Butyloxycarbonyl)-O-(benzyl)]-L-tyrosyl-L-isoleucyl-[N-(dinitrophenyl)]-L-histidyl-L-prolyl-L-alaninmethylester

($R_f^2$-Wert = 0,33) wurde in 10 $cm^3$ einer 8n Lösung von Chlorwasserstoff in Dioxan gelöst und nach 10 Minuten langem Stehenlassen wurde durch Zugabe von wasserfreiem Äther das freie Pentapeptidesterhydrochlorid

[O-(Benzyl)]-L-tyrosyl-L-isoleucyl-[N-(dinitrophenyl)]-L-histidyl-L-prolyl-L-alaninmethylesterhydrochlorid

($R_f^4$-Wert = 0,35) gefällt, abfiltriert und sofort in 20 $cm^3$ Dimethylformamid gelöst. Der pH-Wert der Lösung wurde mit Triäthylamin auf 8 eingestellt und es wurden 1,55 g (4 Millimol)

[N-(tert.-Butyloxycarbonyl)]-L-valinpentafluorphenylester

zugesetzt. Die Lösung wurde bei konstant gehaltenem pH-Wert ½ Stunde stehengelassen, dann wurde das Lösungsmittel abgedampft, der Rückstand in Chloroform gelöst und die Lösung in der üblichen Weise ausgeschüttelt, getrocknet und eingedampft. Der Rückstand wurde mit wasserfreiem Äther verrieben und abfiltriert. Der erhaltene geschützte Hexapeptidester

[N-(tert.-Butyloxycarbonyl)]-L-valyl-[O-(benzyl)]-L-tyrosyl-L-isoleucyl-[N-(dinitrophenyl)]-L-histidyl-L-propyl-L-alaninmethylester

($R_f^2$-Wert = 0,35) wurde in 8 $cm^3$ einer 8n Lösung von Chlorwasserstoff in Dioxan gelöst und nach 20 Minuten langem Stehenlassen wurde durch Zugabe von wasserfreiem Äther das freie Hexapeptidesterhydrochlorid

L-Valyl-[O-(benzyl)]-L-tyrosyl-L-isoleucyl-[N-(dinitrophenyl)]-L-histidyl-L-prolyl-L-alaninmethylesterhydrochlorid

($R_f^5$-Wert = 0,75) gefällt, abfiltriert und mit Äther gewaschen. Dieses Produkt wurde sofort in 20 $cm^3$ Dimethylformamid gelöst, der pH-Wert der Lösung wurde mit Triäthylamin auf 8 eingestellt und es wurden 2,64 g (6 Millimol)

[N-(tert.-Butyloxycarbonyl)-N′-(nitro)]-L-argininpentafluorphenylester

zugesetzt. Die Lösung wurde bei konstant gehaltenem pH-Wert 1 Stunde stehengelassen, dann mit 60 $cm^3$ Chloroform verdünnt, mit einer n Salzsäure und mit Wasser ausgeschüttelt, getrocknet und eingedampft. Der Rückstand wurde in einem Gemisch von Äthanol und Äther im Volumverhältnis von 1 : 3 verrieben, abfiltriert und gewaschen. Der erhaltene geschützte Heptapeptidester

[N-(tert.-Butyloxycarbonyl)-N′-(nitro)]-L-arginyl-L-valyl-[O-(benzyl)]-L-tyrosyl-L-isoleucyl-[N-(dinitrophenyl)]-L-histidyl-L-prolyl-L-alaninmethylester

($R_f^4$-Wert = 0,85) wurde in 10 $cm^3$ einer 8n Lösung von Chlorwasserstoff in Dioxan gelöst und nach 15 Minuten langem Stehenlassen wurde durch Zugabe von wasserfreiem Äther das freie Heptapeptidesterhydrochlorid

[N-(Nitro)]-L-arginyl-L-valyl-[O-(benzyl)]-L-tyrosyl-L-isoleucyl-[N-(dinitrophenyl)]-L-histidyl-L-prolyl-L-alaninmethylester-hydrochlorid

($R_f^4$-Wert = 0,15) gefällt, abfiltriert, gewaschen und sofort in 20 $cm^3$ Dimethylformamid gelöst. Der pH-Wert der Lösung wurde mit Triäthylamin auf 8 eingestellt und es wurden 1,27 g (3,3 Millimol)

[N - (Benzyloxycarbonyl)] - L - sarkosylpentafluor - phenylester

zugesetzt. Die Lösung wurde bei konstant gehaltenem pH-Wert 15 Minuten stehengelassen, dann mit 60 $cm^3$ Chloroform verdünnt und in der üblichen Weise ausgeschüttelt, getrocknet und eingedampft. Der als Rückstand verbliebene geschützte Octapeptidester

[N-(Benzyloxycarbonyl)]-L-sarkosyl-[N-(nitro)]-L-arginyl-L-valyl-[O-(benzyl)]-L-tyrosyl-L-isoleucyl-[N-(dinitrophenyl)]-L-histidyl-L-prolyl-L-alaninmethylester wurde mit 25 $cm^3$ Äthanol verrieben, abfiltriert und gewaschen. So wurden 1,37 g

[N-(Benzyloxycarbonyl)]-L-sarkosyl-[N-(nitro)]-L-arginyl-L-valyl-[O-(benzyl)]-L-tyrosyl-L-isoleucyl-[N-(dinitrophenyl)]-L-histidyl-L-prolyl-L-alaninmethylester

(Schmelzpunkt = 192 bis 196 °C und $R_f^4$-Wert = 0,70) erhalten. (Dies stellt eine Ausbeute von 33% der Theorie, bezogen auf Prolin, dar, was einer durchschnittlichen Ausbeute von 86% der Theorie je Schritt entspricht.)

2. Stufe

L-Sarkosyl-L-arginyl-L-valyl-L-tyrosyl-L-isoleucyl-L-histidyl-L-prolyl-L-alaninmethylester

{Entfernen der Schutzgruppen}

Es wurden 1,37 g (1 Millimol) wie in der vorstehenden 1. Stufe beschrieben erhaltener geschützter Octapeptidester

[N-(Benzyloxycarbonyl)]-L-sarkosyl-[N-(nitro)]-L-arginyl-L-valyl-[O-(benzyl)]-L-tyrosyl-L-isoleucyl-[N-(dinitrophenyl)]-L-histidyl-L-prolyl-L-alaninmethylester

in 3 $cm^3$ Dimethylformamid gelöst und mit 1,9 $cm^3$ 2-Mercaptoäthanol versetzt und nach 1 Stunde langem Stehenlassen wurde durch Zugabe von wasserfreiem Äther 1,2 g (99% der Theorie) des von der Dinitrophenylgruppe befreiten Octapeptidesters

[N-(Benzyloxycarbonyl)]-L-sarkosyl-[N-(nitro)]-L-arginyl-L-valyl-[O-(benzyl)]-L-tyrosyl-L-isoleucyl-L-histidyl-L-prolyl-L-alaninmethylester

($R_f^4$-Wert = 0,20) gefällt, abfiltriert und gewaschen. Dieses Produkt wurde in 20 $cm^3$ eines Gemisches von Methanol, Essigsäure und Wasser im Volumverhältnis von 5 : 1 : 1 gelöst, die Lösung wurde mit 0,6 g von 10 Gew.-% Palladium auf Aktivkohle als Katalysator versetzt und es wurde 20 Stunden lang unter kräftigem Rühren beziehungsweise Schütteln Wasserstoffgas durch das Gemisch geleitet. Nach dem Ende der Reaktion wurde der Katalysator abfiltriert und mit demselben Lösungsmittelgemisch gewaschen und das mit der Waschflüssigkeit vereinigte Filtrat wurde zur Trockne eingedampft und mehrmals mit einem Gemisch von Äthanol und Wasser versetzt und wieder zur Trockne eingedampft. Schliesslich wurde der Rückstand mit einem Gemisch von Äthanol und Äther im Volumverhältnis von 1 : 1 verrieben und abfiltriert. So wurde 0,7 g (75% der Theorie) des freien Octapeptidesters

L-Sarkosyl-L-arginyl-L-valyl-L-tyrosyl-L-isoleucyl-L-histidyl-L-prolyl-L-alaninmethylester

erhalten, welcher dann nach der vor den Beispielen beschriebenen allgemeinen Reinigungsverfahrensweise gereinigt wurde. Der reine

L-Sarkosyl-L-arginyl-L-valyl-L-tyrosyl-L-isoleucyl-L-histidyl-L-prolyl-L-alaninmethylester

hatte die folgenden charakteristischen Eigenschaften:

$R_f^7$-Wert = 0,23.
$R_f^8$-Wert = 0,54.
$R_f^9$-Wert = 0,27.

Aminosäureanalyse:

| | | | |
|---|---|---|---|
| L – Histidin | = | 0,97 [1], | |
| L – Arginin | = | 1,03 [1], | |
| L – Prolin | = | 1,06 [1], | |
| L – Valin | = | 1,03 [1], | |
| L – Isoleucin | = | 0,98 [1], | |
| L – Alanin | = | 1,0 [1], | |
| L – Tyrosin | = | 0,6 [1], | und |
| L – Sarkosin | = | 1,0 [1]. | |

**Patentansprüche für die Vertragstaaten: BE, DE, FR, IT, NL, SE, GB, CH, Li, GB**

1. Peptidester der allgemeinen Formel

X-Arg-Val-Tyr-Ile-His-Pro-Y-O-A I,

worin

X für einen Acylrest einer N-Methylaminosäure oder einer in der α-Stellung eine Aminooxygruppe oder eine Hydroxygruppe aufweisenden aliphatischen Carbonsäure, jeweils in der L-Konfiguration im Falle eines asymmetrischen Kohlenstoffatoms, steht,

Arg einen Rest von L-Arginin bedeutet,

Val einen Rest von L-Valin darstellt,

Tyr für einen Rest von L-Tyrosin steht,

Ile einen Rest von L-Isoleucin bedeutet,

His einen Rest von L-Histidin darstellt,

Pro für einen Rest von L-Prolin steht,

Y einen Rest einer aliphatischen Aminosäure, jeweils in der L-Konfiguration im Falle eines asymmetrischen Kohlenstoffatoms, bedeutet und

A einen Alkylrest mit 1 bis 5 Kohlenstoffatom(en) darstellt,

sowie ihre Säureadditionssalze und pharmazeutisch brauchbaren Komplexe.

2. Peptidester nach Anspruch 1, dadurch gekennzeichnet, dass der Acylrest einer N-Methylaminosäure, für den X stehen kann, der Sarkosylrest ist.

3. Peptidester nach Anspruch 1 oder 2, dadurch gekennzeichnet, dass der Alkylrest, für den A steht, ein solcher mit 1 oder 2, ganz besonders 1, Kohlenstoffatom(en) ist.

4. Peptidester nach Anspruch 1 bis 3, dadurch gekennzeichnet, dass der Rest einer Aminosäure, für den Y steht, ein solcher von L-Isoleucin, L-Threonin, L-Methylthreonin oder L-Alanin ist.

5. L-Sarkosyl-L-arginyl-L-valyl-L-tyrosyl-L-isoleucyl-L-histidyl-L-prolyl-L-isoleucinmethylester.

6. L-α-Aminooxypropionyl-L-arginyl-L-valyl-L-tyrosyl-L-isoleucyl-L-histidyl-L-prolyl-L-isoleucinmethylester.

7. L-Sarkosyl-L-arginyl-L-valyl-L-tyrosyl-L-isoleucyl-L-histidyl-L-prolyl-L-methyltreoninmethylester.

8. Hydroxyacetyl-L-arginyl-L-valyl-L-tyrosyl-L-isoleucyl-L-histidyl-L-prolyl-L-isoleucinmethylester.

9. Hydroxyacetyl-L-arginyl-L-valyl-L-tyrosyl-L-isoleucyl-L-histidyl-L-prolyl-L-methylthreoninmethylester.

10. Hydroxyacetyl-L-arginyl-L-valyl-L-tyrosyl-L-isoleucyl-L-histidyl-L-prolyl-L-threoninmethylester.

11. L-Sarkosyl-L-arginyl-L-valyl-L-tyrosyl-L-isoleucyl-L-histidyl-L-prolyl-L-alaninmethylester.

12. Verfahren zur Herstellung der Verbindungen nach Anspruch 1 bis 11, dadurch gekennzeichnet, dass man in in der Peptidchemie an sich bekannter Weise einen Alkylester einer passenden aliphatischen Aminosäure mit 1 bis 5 Kohlenstoffatomen im Alkylteil mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat desselben kondensiert und gegebenenfalls mit dem erhaltenen am endständigen Stickstoffatom geschützten Peptidzwischenprodukt und dem beziehungsweise den durch etwaige weitere derartige Kondensationen erhaltenen am endständigen Stickstoffatom geschützten weiteren Peptidzwischenprodukt(en) nach in an sich bekannter Weise erfolgendem Entfernen der Schutzgruppe des endständigen Stickstoffatoms eine weitere beziehungsweise weitere Kondensation(en) mit beziehungsweise jeweils mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat desselben vornimmt, wobei man so viele Kondensationen durchführt, wie es zum Einbau aller gewünschten Aminosäureeinheiten erforderlich ist, sowie danach in an sich bekannter Weise vom erhaltenen geschützten Peptidesterderivat die Schutzgruppe des endständigen Stickstoffatoms und die etwaige(n) Schutzgruppe(n) von anderen Stickstoffatomen beziehungsweise Sauerstoffatomen sowie gegebenenfalls die Aminogruppe der gegebenenfalls vorliegenden Aminooxygruppe selektiv schrittweise oder in einem Schritt entfernt, worauf man gegebenenfalls in an sich bekannter Weise den erhaltenen Peptidester der allgemeinen Formel I in ein Säureadditionssalz oder einen Komplex desselben überführt beziehungsweise gegebenenfalls das erhaltene Säureadditionssalz des Peptidesters der allgemeinen Formel I in ein anderes Säureadditionssalz oder in den Peptidester der allgemeinen Formel I überführt.

13. Verfahren nach Anspruch 12, dadurch gekennzeichnet, dass man durch die Kondensation(en) als geschützte Peptidesterderivate solche der allgemeinen Formel

B-X-Arg[C]-Val-Tyr[D]-Ile-His[E]-Pro-Y-O-A    II,

worin

B für eine durch Acidolyse oder katalytische Hydrogenolyse entfernbare Schutzgruppe, insbesondere eine Benzyloxycarbonyl- oder tert.-Butyloxycarbonylgruppe, steht,

C eine Schutzgruppe zum zeitweiligen Schutz der Guanidinogruppe des Restes von Arginin, insbesondere eine Nitro- oder p-Toluolsulfonylgruppe, bedeutet,

D eine Schutzgruppe zum zeitweiligen Schutz der aromatischen Hydroxygruppe des Restes von Tyrosin, insbesondere eine, gegebenenfalls substituierte, Benzylgruppe, darstellt,

E für eine Schutzgruppe zum zeitweiligen Schutz der Imidazolgruppe des Restes von Histidin, insbesondere eine Dinitrophenylgruppe, steht und

X, Arg, Val, Tyr, Ile, His, Pro, Y und A wie in den Ansprüchen 1 bis 4 festgelegt sind,

aufbaut.

14. Verfahren nach Anspruch 12 oder 13 zur Herstellung von Peptidestern der allgemeinen Formel I, bei welchen X für einen Acylrest einer in der α-Stellung eine Aminooxygruppe aufweisenden aliphatischen Carbonsäure steht, dadurch gekennzeichnet, dass man durch die Kondensation(en) die entsprechenden geschützten Peptidester der allgemeinen Formel II, bei welchen X ebenfalls für einen Acylrest einer in der α-Stellung eine Aminooxygruppe aufweisenden aliphatischen Carbonsäure steht, aufbaut und das Abspalten der Schutzgruppe, für welche B steht, sowie gegebenenfalls der anderen durch Acidolyse abspaltbaren Schutzgruppe(n) von ihnen durch Acidolyse, insbesondere durch Behandeln mit Fluorwasserstoffsäure, durchführt.

15. Verfahren nach Anspruch 12 oder 13 zur Herstellung von Peptidestern der allgemeinen Formel I, bei welchen X für einen Acylrest einer in der α-Stellung eine Hydroxygruppe aufweisenden aliphatischen Carbonsäure steht, dadurch gekennzeichnet, dass man durch die Kondensation(en) die entsprechenden Peptidester der allgemeinen Formel II, bei welchen jedoch X für einen Acylrest einer in der α-Stellung eine Aminooxygruppe aufweisenden aliphatischen Carbonsäure steht, aufbaut und das Abspalten der Schutzgruppe, für welche B steht, sowie gegebenenfalls der anderen durch katalytische Hydrogenolyse abspaltbaren Schutzgruppe(n) von ihnen durch katalytische Hydrierung unter gleichzeitiger Abspaltung der Aminogruppe der genannten α-Aminooxygruppe durchführt.

16. Arzneimittel beziehungsweise Diagnostica, gekennzeichnet durch einen Gehalt an 1 oder mehr Verbindungen nach Anspruch 1 bis 11 als Wirkstoff beziehungsweise Wirkstoffen, zweckmässigerweise zusammen mit üblichen pharmazeutischen Konfektionierungsmitteln.

**Patentansprüche für den Vertragstaat: AT**

1. Verfahren zur Herstellung von Peptidestern der allgemeinen Formel

X-Arg-Val-Tyr-Ile-His-Pro-Y-O-A I,

worin

X für einen Acylrest einer N-Methylaminosäure oder einer in der α-Stellung eine Aminooxygruppe oder eine Hydroxygruppe aufweisenden aliphatischen Carbonsäure, jeweils in der L-Konfiguration im Falle eines asymmetrischen Kohlenstoffatoms, steht,

Arg einen Rest von L-Arginin bedeutet,

Val einen Rest von L-Valin darstellt,

Tyr für einen Rest von L-Tyrosin steht,

Ile einen Rest von L-Isoleucin bedeutet,

His einen Rest von L-Histidin darstellt,

Pro für einen Rest von L-Prolin steht,

Y einen Rest einer aliphatischen Aminosäure, jeweils in der L-Konfiguration im Falle eines asymmetrischen Kohlenstoffatoms, bedeutet und

A einen Alkylrest mit 1 bis 5 Kohlenstoffatom(en) darstellt,

sowie ihren Säureadditionssalzen und pharmazeutisch brauchbaren Komplexen, dadurch gekennzeichnet, dass man in in der Peptidchemie an sich bekannter Weise einen Alkylester einer passenden aliphatischen Aminosäure mit 1 bis 5 Kohlenstoffatomen im Alkylteil mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/ oder Sauerstoffatom(en) eine beziehungsweise mehrere Schutzgruppe(n) aufweisenden Peptidfragment beziehungsweise einem Esterderivat desselben kondensiert und gegebenenfalls mit dem erhaltenen am endständigen Stickstoffatom geschützten Peptidzwischenprodukt und dem beziehungsweise den durch etwaige weitere derartige Kondensationen erhaltenen am endständigen Stickstoffatom geschützten weiteren Peptidzwischenprodukt(en) nach in an sich bekannter Weise erfolgendem Entfernen der Schutzgruppe des endständigen Stickstoffatoms eine weitere beziehungsweise weitere Kondensation(en) mit beziehungsweise jeweils mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat desselben vornimmt, wobei man so viele Kondensationen durchführt, wie es zum Einbau aller gewünschten Aminosäureeinheiten erforderlich ist, sowie danach in an sich bekannter Weise vom erhaltenen geschützten Peptidesterderivat die Schutzgruppe des endständigen Stickstoffatoms und die etwaige(n) Schutzgruppe(n) von anderen Stickstoffatomen beziehungsweise Sauerstoffatomen sowie gegebenenfalls die Aminogruppe der gegebenenfalls vorliegenden Aminooxygruppe selektiv schrittweise oder in einem Schritt entfernt, worauf man gegebenenfalls in an sich bekannter Weise den erhaltenen Peptidester der allgemeinen Formel I in ein Säureadditionssalz oder einen Komplex desselben überführt beziehungsweise gegebenenfalls das erhaltene Säureadditionssalz des Peptidesters der allgemeinen Formel I in ein anderes Säureadditionssalz oder in den Peptidester der allgemeinen Formel I überführt.

2. Verfahren nach Anspruch 1 zur Herstellung von Peptidestern der allgemeinen Formel I, bei welchen X für einen Acylrest einer in der α-Stellung eine Aminooxygruppe aufweisenden aliphatischen Carbonsäure steht, dadurch gekennzeichnet, dass man durch die Kondensation(en) die entsprechenden geschützten Peptidester der allgemeinen Formel II, bei welchen X ebenfalls für einen Acylrest einer in der α-Stellung eine Aminooxygruppe aufweisenden aliphatischen Carbonsäure steht, aufbaut und das Abspalten der Schutzgruppe, für welche B steht, sowie gegebenenfalls der anderen durch Acidolyse abspaltbaren Schutzgruppe(n) von ihnen durch Acidolyse, insbesondere durch Behandeln mit Fluorwasserstoffsäure, durchführt.

3. Verfahren nach Anspruch 1 zur Herstellung von Peptidestern der allgemeinen Formel I, bei welchen X für einen Acylrest einer in der α-Stellung eine Hydroxygruppe aufweisenden aliphatischen Carbonsäure steht, dadurch gekennzeichnet, dass man durch die Kondensation(en) die entsprechenden Peptidester der allgemeinen Formel II, bei welchen jedoch X für einen Acylrest einer in der α-Stellung eine Aminooxygruppe aufweisenden aliphatischen Carbonsäure steht, aufbaut und das Abspalten der Schutzgruppe, für welche B steht, sowie gegebenenfalls der anderen durch katalytische Hydrogenolyse abspaltbaren Schutzgruppe(n) von ihnen durch katalytische Hydrierung unter gleichzeitiger Abspaltung der Aminogruppe der genannten α-Aminooxygruppe durchführt.

4. Verfahren nach Anspruch 1 bis 3, dadurch gekennzeichnet, dass man als Peptidester solche, bei welchen der Acylrest einer N-Methylaminosäure, für den X stehen kann, der Sarkosylrest ist, herstellt.

5. Verfahren nach Anspruch 1 bis 4, dadurch gekennzeichnet, dass man als Peptidester solche, bei welchen der Alkylrest, für den A steht, ein solcher mit 1 oder 2, ganz besonders 1, Kohlenstoffatom(en) ist, herstellt.

6. Verfahren nach Anspruch 1 bis 5, dadurch gekennzeichnet, dass man als Peptidester solche, bei welchen der Rest einer Aminosäure, für den Y steht, ein solcher von L-Isoleucin, L-Threonin, L-Methylthreonin oder L-Alanin ist, herstellt.

7. Verfahren nach Anspruch 1 bis 6, dadurch gekennzeichnet, dass man als Peptidester L-Sarkosyl-L-arginyl-L-valyl-L-tyrosyl-L-isoleucyl-L-histidyl-L-prolyl-L-isoleucinmethylester,
L-α-Aminooxypropionyl-L-arginyl-L-valyl-L-tyrosyl-L-isoleucyl-L-histidyl-L-prolyl-L-isoleucinmethylester
L-Sarkosyl-L-arginyl-L-valyl-L-tyrosyl-L-isoleucyl-L-histidyl-L-prolyl-L-methylthreoninmethylester,
Hydroxyacetyl-L-arginyl-L-valyl-L-tyrosyl-L-isoleucyl-L-histidyl-L-prolyl-L-isoleucinmethylester,
Hydroxyacetyl-L-arginyl-L-valyl-L-tyrosyl-L-isoleucyl-L-histidyl-L-prolyl-L-methylthreoninmethylester,
Hydroxyacetyl-L-arginyl-L-valyl-L-tyrosyl-L-isoleucyl-L-histidyl-L-prolyl-L-threoninmethylester oder
L-Sarkosyl-L-arginyl-L-valyl-L-tyrosyl-L-isoleucyl-L-histidyl-L-prolyl-L-alaninmethylester
herstellt.

8. Verfahren nach Anspruch 1 bis 7, dadurch gekennzeichnet, dass man durch die Kondensation(en) als geschützte Peptidesterderivate solche der allgemeinen Formel

B-X-Arg[C]-Val-Tyr[D]-Ile-His[E]-Pro-Y-O-A II,

worin

B für eine durch Acidolyse oder katalytische Hydrogenolyse entfernbare Schutzgruppe, insbesondere eine Benzyloxycarbonyl- oder tert.-Butyloxycarbonylgruppe, steht,

C eine Schutzgruppe zum zeitweiligen Schutz der Guanidinogruppe des Restes von Arginin, insbesondere eine Nitro- oder p-Toluolsulfonylgruppe, bedeutet,

D eine Schutzgruppe zum zeitweiligen Schutz der aromatischen Hydroxygruppe des Restes von Tyrosin, insbesondere eine, gegebenenfalls substituierte, Benzylgruppe, darstellt,

E für eine Schutzgruppe zum zeitweiligen Schutz der Imidazolgruppe des Restes von Histidin, insbesondere eine Dinitrophenylgruppe, steht und

X, Arg, Val, Tyr, Ile, His, Pro, Y und A wie in den Ansprüchen 1 bis 4 festgelegt sind,
aufbaut.

**Claims for the Contracting states BE, CH, DE, FR, GB, IT, Li, NL, SE**

1. Peptide esters having the general formula

X-Arg-Val-Tyr-Ile-His-Pro-Y-O-A I,

wherein

X represents an acyl radical of a N-methylamino acid or of an aliphatic carboxylic acid having in the α-position an aminooxy group or a hydroxyl group, each in the L-configuration in case of an asymetrical carbon atom,

Arg means a radical of L-arginine,
Val represents a radical of L-valine,
Tyr represents a radical of L-tyrosine,
Ile means a radical of L-isoleucine,
His represents a radical of L-histidine,
Pro represents a radical of L-proline,
Y means a radical of an aliphatic amino acid, each in the L-configuration in case of an asymetrical carbon atom, and
A represents an alkyl radical having from 1 to 5 carbon atom(s),

as well as their acid addition salts and pharmaceutically useful complexes.

2. Peptide esters according to claim 1, characterized in that the acyl radical of a N-methylamino acid which can be represented by X is the sarcosyl radical.

3. Peptide esters according to claim 1 or 2, characterized in that the alkyl radical which may be represented by A is such having 1 or 2, most particularly 1, carbon atom(s).

4. Peptide esters according to claims 1 to 3, characterized in that the radical of an amino acid which may be represented by Y is such of L-isoleucine, L-threonine, L-methylthreonine or L-alanine.

5. L-Sarcosyl-L-arginyl-L-valyl-L-tyrosyl-L-isoleucyl-L-histidyl-L-prolyl-L-isoleucinemethylester.

6. L-α-Aminooxypropionyl-L-arginyl-L-valyl-L-tyrosyl-L-isoleucyl-L-histidyl-L-prolyl-L-isoleucinemethylester.

7. L-Sarcosyl-L-arginyl-L-valyl-L-tyrosyl-L-isoleucyl-L-histidyl-L-prolyl-L-methylthreoninemethylester.

8. Hydroxyacetyl-L-arginyl-L-valyl-L-tyrosyl-L-isoleucyl-L-histidyl-L-prolyl-L-isoleucinemethylester.

9. Hydroxyacetyl-L-arginyl-L-valyl-L-tyrosyl-L-isoleucyl-L-histidyl-L-prolyl-L-methylthreoninemethylester.

10. Hydroxyacetyl-L-arginyl-L-valyl-L-tyrosyl-L-isoleucyl-L-histidyl-L-prolyl-L-threoninemethylester.

11. L-Sarcosyl-L-arginyl-L-valyl-L-tyrosyl-L-isoleucyl-L-histidyl-L-prolyl-L-alaninemethylester.

12. A process for the preparation of the compounds according to claims 1 to 11, characterized in that one condenses in a manner known per se in the peptide chemistry a proper alkyl ester of a proper aliphatic amino acid having from 1 to 5 carbon atoms in the alkyl part with the amino acid

to be incorporated successively and having on its terminal nitrogen atom a splittable-off protective group and, as far as necessary, on another nitrogen atom being optionally present another splittable-off protective group and/or with the peptide fragment or an ester derivative of it, respectively, successively to be incorporated and having on its terminal nitrogen atom a splittable-off protective group and, as far as necessary, on one or several protective group(s) and, if desired, one carries out with the obtained peptide intermediate protected on the terminal nitrogen atom and with the further peptide intermediate(s) obtained by possible further condensations of this type and protected on the terminal nitrogen atom after having removed the protective group of the terminal nitrogen atom one carries out a further condensation or further condensations, respectively, with the amino acid or with the respective amino acid, respectively, successively to be incorporated and having on its terminal nitrogen atom a splittable-off protective group and, as far as necessary, on another nitrogen atom or oxygen atom optionally present another splittable-off protective group and/or with the peptide fragment or an ester derivative of it, respectively, successively to be incorporated and having on its terminal nitrogen atom a splittable-off protective group and, as far as necessary, on one or several other nitrogen and/or oxygen atom(s) optionally present one or several protective group(s) accomplishing as much condensations as are necessary for incorporating all desired amino acid units as well as thereafter in a manner known per se selectively step by step or in one step one removes from the obtained protected peptide ester derivative the protective group of the terminal nitrogen atom and the possible protective group(s) of other nitrogen atoms or oxygen atoms, respectively, as well as, if desired, the amino group of the aminooxy group optionally present, whereafter, if desired, in a manner known per se one converts the obtained peptide ester of the general formula I into an acid addition salt or into a complex of it or, if desired, the obtained acid addition salt of the peptide ester of the general formula I into another acid addition salt or in the peptide ester of the general formula I, respectively.

13. A process according to claim 12, characterized in that one synthesizes by the condensation(s) as protected peptide ester derivatives such having the general formula

B-X-Arg[C]-Val-Tyr[D]-Ile-His[E]-
Pro-Y-O-A II,

wherein

B represents a protective group removable by acidolysis or catalytic hydrogenolysis, particularly a benzyloxycarbonyl or tert.-butyloxycarbonyl group,

C means a protective group for the temporary protection of the guanidino group of the radical of arginine, particularly a nitro or p-toluenesulfonyl group,

D represents a protective group for the temporary protection of the aromatic hydroxyl group of the radical of tyrosine, particularly a benzyl group, optionally substituted,

E represents a protective group for the temporary protection of the imidazole group of the radical of histidine, particularly a dinitrophenyl group, and

X, Arg, Val, Tyr, Ile, His, Pro, Y and A are as defined in claims 1 to 4.

14. A process according to claim 12 or 13 for the preparation of peptide esters of the general formula I in which X represents an acyl radical of an aliphatic carboxylic acid having in the α-position an aminooxy group, characterized in that one synthesizes by the condensation(s) the corresponding protective peptide esters of the general formula II, in which X likewise represents an acyl radical of an aliphatic carboxylic acid having an aminooxy group in the α-position and one carries out the splitting-off of the protective group represented by B as well as, if desired, of the other protective group(s) splittable-off by acidolysis from them by acidolysis, particularly by treatment with hydrofluoric acid.

15. A process according to claim 12 or 13 for the preparation of peptide esters of the general formula I in which X represents an acyl radical of an aliphatic carboxylic acid having a hydroxyl group in the α-position, characterized in that one synthesizes by the condensation(s) the corresponding peptide esters of the general formula II in which, however, X represents an acyl radical of an aliphatic carboxylic acid having an aminooxy group in the α-position and one carries out the splitting-off of the protective group represented by B as well, if desired, of the other protective group(s) splittable-off by catalytic hydrogenolysis from them by catalytic hydrogenation with simultaneous splitting-off of the amino group of the said α-aminooxy group.

16. Medicaments or diagnostic agents, respectively, characterized by a content of 1 or more compounds according to claims 1 to 11 as an active principle or active principles, respectively, usefully together with usual pharmaceutical processing agents.

**Claims for the Contracting state: AT**

1. A process for the preparation of peptide esters having the general formula

X-Arg-Val-Tyr-Ile-His-Pro-Y-O-A I,

wherein

X represents an acyl radical of a N-methylamino acid or of an aliphatic carboxylic acid having in the α-position an aminooxy group or a hydroxyl group, each in the L-configuration in case of an asymetrical carbon atom,

Arg means a radical of L-arginine,
Val represents a radical of L-valine,
Tyr represents a radical of L-tyrosine,
Ile means a radical of L-isoleucine,
His represents a radical of L-histidine,
Pro represents a radical of L-proline,

Y means a radical of an aliphatic amino acid, each in the L-configuration in case of an asymetrical carbon atom, and

A represents an alkyl radical having from 1 to 5 carbon atom(s),

as well as their acid addition salts and pharmaceutically useful complexes, characterized in that one condenses in a manner known per se in the peptide chemistry a proper alkyl ester of a proper aliphatic amino acid having from 1 to 5 carbon atoms in the alkyl part with the amino acid to be incorporated successively and having on its terminal nitrogen atom a splittable-off protective group and, as far as necessary, on another nitrogen atom being optionally present another splittable-off protective group and/or with the peptide fragment or an ester derivative of it, respectively, successively to be incorporated and having on its terminal nitrogen atom a splittable-off protective group and, as far as necessary, on one or several protective group(s) and, if desired, one carries out with the obtained peptide intermediate protected on the terminal nitrogen atom and with the further peptide intermediate(s) obtained by possible further condensations of this type and protected on the terminal nitrogen atom after having removed the protective group of the terminal nitrogen atom one carries out a further condensation or further condensations, respectively, with the amino acid or with the respective amino acid, respectively, successively to be incorporated and having on its terminal nitrogen atom a splittable-off protective group and, as far as necessary, on another nitrogen atom or oxygen atom optionally present another splittable-off protective group and/or with the peptide fragment or an ester derivative of it, respectively, successively to be incorporated and having on its terminal nitrogen atom a splittable-off protective group and, as far as necessary, on one or several other nitrogen and/or oxygen atom(s) optionally present one or several protective group(s) accomplishing as much condensations as are necessary for incorporating all desired amino acid units as well as thereafter in a manner known per se selectively step by step or in one step one removes from the obtained protected peptide ester derivative the protective group of the terminal nitrogen atom and the possible protective group(s) of other nitrogen atoms or oxygen atoms, respectively, as well as, if desired, the amino group of the aminooxy group optionally present, whereafter, if desired, in a manner known per se one converts the obtained peptide ester of the general formula I into an acid addition salt or into a complex of it or, if desired, the obtained acid addition salt of the peptide ester of the general formula I into another acid addition salt or in the peptide ester of the general formula I, respectively.

2. A process according to claim 1 for the preparation of peptide esters of the general formula I in which X represents an acyl radical of an aliphatic carboxylic acid having in the α-position an aminooxy group, characterized in that one synthesizes by the condensation(s) the corresponding protective peptide esters of the general formula II, in which X likewise represents an acyl radical of an aliphatic carboxylic acid having an aminooxy group in the α-position and one carries out the splitting-off of the protective group represented by B as well as, if desired, of the other protective group(s) splittable-off by acidolysis from them by acidolysis, particularly by treatment with hydrofluoric acid.

3. A process according to claim 1 for the preparation of peptide esters of the general formula I in which X represents an acyl radical of an aliphatic carboxylic acid having a hydroxyl group in the α-position, characterized in that one synthesizes by the condensation(s) the corresponding peptide esters of the general formula II in which, however, X represents an acyl radical of an aliphatic carboxylic acid having an aminooxy group in the α-position and one carries out the splitting-off of the protective group represented by B as well, if desired, of the other protective group(s) splittable-off by catalytic hydrogenolysis from them by catalytic hydrogenation with simultaneous splitting-off of the amino group of the said α-aminooxy group.

4. A process according to claims 1 to 3, characterized in that one prepares as peptide esters such ones in which the acyl radical of a N-methylamino acid which can be represented by X is the sarcosyl radical.

5. A process according to claims 1 to 4, characterized in that one prepares as peptide esters such ones in which the alkyl radical which may be represented by A is such having 1 or 2, most particularly 1, carbon atom(s).

6. A process according to claims 1 to 5, characterized in that one prepares as peptide esters such ones in which the radical of an amino acid which may be represented by Y is such of L-isoleucine, L-threonine, L-methyl-threonine or L-alanine.

7. A process according to claims 1 to 6, characterized in that one prepares

L-sarcosyl-L-arginyl-L-valyl-L-tyrosyl-L-isoleucyl-L-histidyl-L-prolyl-L-isoleucine-methylester,

L-α-aminooxypropionyl-L-arginyl-L-valyl-L-tyrosyl-L-isoleucyl-L-histidyl-L-prolyl-L-isoleucinemethylester,

L-sarcosyl-L-arginyl-L-valyl-L-tyrosyl-L-isoleucyl-L-histidyl-L-prolyl-L-methylthreoninemethylester,

hydroxyacetyl-L-arginyl-L-valyl-L-tyrosyl-L-isoleucyl-L-histidyl-L-prolyl-L-isoleucinemethylester,

hydroxyacetyl-L-arginyl-L-valyl-L-tyrosyl-L-isoleucyl-L-histidyl-L-prolyl-L-methylthreoninemethylester,

hydroxyacetyl-L-arginyl-L-valyl-L-tyrosyl-L-isoleucyl-L-histidyl-L-prolyl-L-threoninemethylester or

L-sarcosyl-L-arginyl-L-valyl-L-tyrosyl-L-isoleucyl-L-histidyl-L-prolyl-L-alaninemethylester.

8. A process according to claims 1 to 7, characterized in that one synthesizes by the condensation(s) as protected peptide ester derivatives such having the general formula

B-X-Arg[C]-Val-Tyr[D]-Ile-His[E]-Pro-Y-O-A II,

wherein

B represents a protective group removable by acidolysis or catalytic hydrogenolysis, particularly a benzyloxycarbonyl or tert.-butyloxycarbonyl group,

C means a protective group for the temporary protection of the guanidino group of the radical of arginine, particularly a nitro or p-toluenesulfonyl group,

D represents a protective group for the temporary protection of the aromatic hydroxyl group of the radical of tyrosine, particularly a benzyl group, optionally substituted,

E represents a protective group for the temporary protection of the imidazole group of the radical of histidine, particularly a dinitrophenyl group, and

X, Arg, Val, Tyr, Ile, His, Pro, Y and A are as defined in claims 1 or 4 to 6.

**Revendications pour les États contractants: BE, CH, DE, FR, GB, IT, Li, NL, SE**

1. Esters de peptide de formule générale:

X-Arg-Val-Tyr-Ile-His-Pro-Y-O-A (I)

dans laquelle:

X représente un radical acyle d'un N-méthylaminoacide ou d'un acide carboxylique aliphatique présentant en position α un groupe aminoxy ou un groupe hydroxyle, chaque fois en configuration L dans le cas d'un atome de carbone asymétrique,

Arg représente un radical de L-arginine,
Val représente un radical de L-valine,
Tyr représente un radical de L-tyrosine,
Ile représente un radical de L-isoleucine,
His représente un radical de L-histidine,
Pro représente un radical de L-proline,

Y représente un radical d'un aminoacide aliphatique, chaque fois en configuration L dans le cas d'un atome de carbone asymétrique, et

A représente un radical alkyle de 1 à 5 atomes de carbone, ainsi que leurs sels d'addition d'acide et leurs complexes utilisables pharmaceutiquement.

2. Esters de peptide selon la revendication 1, caractérisés par le fait que le radical acyle N-méthylaminoacide que peut représenter X est le radical sarcosyle.

3. Esters de peptide selon l'une des revendications 1 et 2, caractérisés par le fait que le radical alkyle que représente A est un radical contenant 1 ou 2 atomes de carbone, tout particulièrement 1.

4. Esters de peptide selon l'une des revendications 1 à 3, caractérisés par le fait que le radical d'aminoacide que représente Y est un radical de L-isoleucine, de L-thréonine, de L-méthylthréonine ou de L-alanine.

5. Ester méthylique de L-sarcosyl-L-arginyl-L-valyl-L-tyrosyl-L-isoleucyl-L-histidyl-L-prolyl-L-isoleucine.

6. Ester méthylique de L-α-aminooxypropionyl-L-arginyl-L-valvyl-L-tyrosyl-L-isoleucyl-L-histidyl-L-prolyl-L-isoleucine.

7. Ester méthylique de L-sarcosyl-L-arginyl-L-valyl-L-tyrosyl-L-isoleucyl-L-histidyl-L-prolyl-L-méthylthréonine.

8. Ester méthylique d'hydroxyacétyl-L-arginyl-L-valyl-L-tyrosyl-L-isoleucyl-L-histidyl-L-prolyl-L-isoleucine.

9. Ester méthylique d'hydroxyacétyl-L-arginyl-L-valyl-L-tyrosyl-L-isoleucyl-L-histidyl-L-prolyl-L-méthylthréonine.

10. Ester méthylique d'hydroxyacétyl-L-arginyl-L-valyl-L-tyrosyl-L-isoleucyl-L-histidyl-L-prolyl-L-thréonine.

11. Ester méthylique de L-sarcosyl-L-arginyl-L-valvyl-L-tyrosyl-L-isoleucyl-L-histidyl-L-prolyl-L-alanine.

12. Procédé de préparation des composés selon les revendications 1 à 11, caractérisé par le fait que, de manière en elle-même connue dans la chimie des peptides, on condense un ester alkylique d'un aminoacide aliphatique approprié contenant 1 à 5 atomes de carbone dans le fragment alkyle avec l'aminoacide à incorporer ensuite, présentant sur son atome d'azote terminal un groupe protecteur séparable et dans la mesure où c'est nécessaire, sur un autre atome d'azote éventuellement présente ou sur un atome d'oxygène, un autre groupe protecteur séparable et/ou avec le fragment de peptide à incorporer ensuite, présentant sur son atome d'azote terminal un groupe protecteur séparable et dans la mesure où c'est nécessaire, sur un ou plusieurs autres atomes d'azote et/ou atomes d'oxygène éventuellement présents, un ou plusieurs groupes protecteurs, ou avec un dérivé ester de ce fragment et qu'éventuellement, avec le peptide intermédiaire obtenu, protégé sur l'atome d'azote terminal et avec le ou les autres peptides intermédiaires obtenus par d'autres condensations éventuelles de ce genre et protégés sur l'atome d'azote terminal, après avoir éliminé de manière en elle-même connue, le groupe protecteur de l'atome d'azote terminal, on opère une autre ou d'autres condensations avec l'aminoacide ou chaque aminoacide à incorporer ensuite, présentant sur son atome d'azote terminal un groupe protecteur séparable et dans la mesure où c'est nécessaire, sur un autre atome d'azote ou atome d'oxygène éventuellement présent, un autre groupe protecteur séparable, et/ou avec le fragment de peptide à incorporer ensuite, présentant sur son atome d'azote terminal un groupe protecteur séparable et dans la mesure où

c'est nécessaire, sur un ou plusieurs autres atomes d'azote et/ou d'oxygène éventuellement présents, un ou plusieurs groupes protecteurs, ou avec un dérivé ester de ce fragment, en effectuant autant de condensations qu'il est nécessaire pour incorporer toutes les unités aminoacide désirées, et qu'ensuite, de manière en elle-même connue, du dérivé ester de peptide protégé obtenu, on élimine le groupe protecteur de l'atome d'azote terminal et le ou les groupes protecteurs éventuels d'autres atomes d'azote ou d'atomes d'oxygène et éventuellement aussi le groupe amine du groupe aminooxy éventuellement présent, sélectivement par étapes ou, en une seule étape, après quoi, éventuellement, de manière en elle-même connue, on convertit l'ester de peptide obtenu de formule générale I en un sel d'addition d'acide ou un complexe de celui-ci, ou qu'éventuellement on convertit le sel d'addition d'acide obtenu de l'ester de peptide de formule générale I en un autre sel d'addition d'acide ou en ester de peptide de formule générale I.

13. Procédé selon la revendication 12, caractérisé par le fait que par la ou les condensations, on constitue, comme dérivés protégés d'esters de peptide, des dérivés de formule générale:

B-X-Arg(C)-Val-Tyr(D)-Ile-His(E)-Pro-Y-O-A (II)

dans laquelle:

B représente un groupe protecteur séparable par acidolyse ou par hydrogénolyse catalytique, en particulier un groupe benzyloxycarbonyle ou tertiobutyloxycarbonyle,

C représente un groupe protecteur pour la protection temporaire du groupe guanidine du radical de l'arginine, en particulier un groupe nitro ou p-toluènesulfonyle,

D représente un groupe protecteur pour la protection temporaire du groupe hydroxyle aromatique du radical de la tyrosine, en particulier un groupe benzyle éventuellement substitué,

E représente un groupe protecteur pour la protection temporaire du groupe imidazole du radical de l'histidine, en particulier un groupe dinitrophényle, et

X, Arg, Val, Tyr, Ile His, Pro, Y et A sont tels que définis aux revendications 1 à 4.

14. Procédé selon l'une des revendications 12 et 13, pour la préparation d'esters de peptide de formule générale I dans lesquels X représente un radical acyle d'un acide carboxylique aliphatique présentant en position α un groupe aminooxy, caractérisé par le fait que par la ou les condensations, on constitue les esters de peptide protégés correspondants de formule générale II, dans lesquels X représente également un radical acyle d'un acide carboxylique aliphatique présentant en position α un groupe aminooxy, et que l'on effectue la séparation par acidolyse du groupe protecteur que représente B ainsi qu'éventuellement du ou des autres groupes protecteurs séparables par acidolyse, en particulier en traitant par l'acide fluorhydrique.

15. Procédé selon l'une des revendications 12 et 13, pour la préparation d'esters de peptide de formule générale I, dans lesquels X représente un radical acyle d'un acide carboxylique aliphatique présentant en position α un groupe hydroxyle, caractérisé par le fait que par la ou les condensations, on constitue les esters de peptide correspondants de formule générale I, mais dans lesquels X représente un radical acyle d'un acide carboxylique aliphatique présentant en position α un groupe aminooxy et que l'on effectue la séparation par hydrogénation catalytique du groupe protecteur que représente B, ainsi qu'éventuellement du ou des autres groupes protecteurs séparables par hydrogénolyse catalytique, en séparant simultanément le groupe amine du groupe α-aminooxy mentionné.

16. Médicaments ou agents de diagnostic, caractérisés par une teneur en un ou plusieurs composés selon les revendications 1 à 11 comme substance(s) active(s), de préférence en même temps que des agents de conditionnement pharmaceutiques usuels.

**Revendications pour l'État contractant: AT**

1. Procédé de préparation d'esters de peptide de formule générale:

X-Arg-Val-Tyr-Ile-His-Pro-Y-O-A (I)

dans laquelle:

X représente un radical acyle d'un N-méthylaminoacide ou d'un acide carboxylique aliphatique présentant en position α un groupe aminoxy ou un groupe hydroxyle, chaque fois en configuration L dans le cas d'un atome de carbone asymétrique,

Arg représente un radical de L-arginine,
Val représente un radical de L-valine,
Tyr représente un radical de L-tyrosine,
Ile représente un radical de L-isoleucine,
His représente un radical de L-histidine,
Pro représente un radical de L-proline,

Y représente un radical d'un aminoacide aliphatique, chaque fois en configuration L dans le cas d'un atome de carbone asymétrique, et

A représente un radical alkyle de 1 à 5 atomes de carbone, ainsi que leurs sels d'addition d'acide et leurs complexes utilisables pharmaceutiquement, caractérisé par le fait que, de manière en elle-même connue dans la chimie des peptides, on condense un ester alkylique d'un aminoacide aliphatique approprié contenant 1 à 5 atomes de carbone dans le fragment alkyle avec l'aminoacide à incorporer ensuite, présentant sur son atome d'azote terminal un groupe protecteur séparable et dans la mesure où c'est nécessaire, sur un autre atome d'azote éventuellement présente ou sur un atome d'oxygène, un autre groupe protecteur séparable et/ou avec le fragment de peptide à incorporer ensuite, présentant sur son atome d'azote terminal un groupe protecteur séparable et dans la mesure où c'est nécessaire, sur un ou plusieurs autres atomes d'azote et/ou atomes d'oxygène éventuellement présents, un ou plusieurs groupes protecteurs, ou avec un dérivé ester de ce fragment et qu'éventuellement, avec le peptide intermédiaire obtenu, protégé sur l'atome d'azote terminal et avec le ou les autres peptides intermédiaires obtenus par d'autres condensations éventuelles de ce genre et protégés sur l'atome d'azote terminal, après avoir éliminé de manière en elle-même connue, le groupe protecteur de l'atome d'azote terminal, on opère un autre ou d'autres condensations avec l'aminoacide ou chaque aminoacide à incorporer ensuite, présentant sur son atome d'azote terminal un groupe protecteur séparable et dans la mesure où c'est nécessaire, sur un autre atome d'azote ou atome d'oxygène éventuellement présent, un autre groupe protecteur séparable, et/ou avec le fragment de peptide à incorporer ensuite, présentant sur son atome d'azote terminal un groupe protecteur séparable et dans la mesure où c'est nécessaire, sur un ou plusieurs autres atomes d'azote et/ou d'oxygène éventuellement présents, un ou plusieurs groupes protecteurs, ou avec un dérivé ester de ce fragment, en effectuant autant de condensations qu'il est nécessaire pour incorporer toutes les unités aminoacide désirées, et qu'ensuite, de manière en elle-même connue, du dérivé ester de peptide protégé obtenu, on élimine le groupe protecteur de l'atome d'azote terminal et le ou les groupes protecteurs éventuels d'autres atomes d'azote ou d'atomes d'oxygène et éventuellement aussi le groupe amine du groupe aminooxy éventuellement présent, sélectivement par étapes ou en une seule étape, après quoi, éventuellement, de manière en elle-même connue, on convertit l'ester de peptide obtenu de formule générale I en un sel d'addition d'acide ou un complexe de celui-ci, ou qu'éventuellement on convertit le sel d'addition d'acide obtenu de l'ester de peptide de formule générale I en un autre sel d'addition d'acide ou en ester de peptide de formule générale I.

2. Procédé selon la revendication 1 pour la préparation d'esters de peptide de formule générale I dans lesquels X représente un radical acyle d'un acide carboxylique aliphatique présentant en position α un groupe aminooxy, caractérisé par le fait que par la ou les condensations, on constitue les esters de peptide protégés correspondants de formule générale II, dans lesquels X représente également un radical acyle d'un acide carboxylique aliphatique présentant en position α un groupe aminooxy, et que l'on effectue la séparation par acidolyse du groupe protecteur que représente B ainsi qu'éventuellement du ou des autres groupes protecteurs séparables par acidolyse, en particulier en traitant par l'acide fluorhydrique.

3. Procédé selon la revendication 1 pour la préparation d'esters de peptide de formule générale I, dans lesquels X représente un radical acyle d'un acide carboxylique aliphatique présentant en position α un groupe hydroxyle, caractérisé par le fait que par la ou les condensations, on constitue les esters de peptide correspondants de formule générale I, mais dans lesquels X représente un radical acyle d'un acide carboxylique aliphatique présentant en position α un groupe aminooxy et que l'on effectue la séparation par hydrogénation catalytique du groupe protecteur que représente B, ainsi qu'éventuellement du ou des autres groupes protecteurs séparables par hydrogénolyse catalytique, en séparant simultanément le groupe amine du groupe α-aminooxy mentionné.

4. Procédé selon les revendications 1 à 3, caractérisé par le fait qu'on prépare comme esters de peptide telles dans lesquelles le radical acyle N-méthylaminoacide que peut représenter X est le radical sarcosyle.

5. Procédé selon les revendications 1 à 4, caractérisé par le fait qu'on prépare comme esters de peptide telles dans lesquelles le radical alkyle que représente A est un radical contenant 1 ou 2 atomes de carbone, tout particulièrement 1.

6. Procédé selon les revendications 1 à 5, caractérisé par le fait qu'on prépare comme esters de peptide telles dans lesquelles le radical d'aminoacide que représente Y est un radical de L-isoleucine, de L-thréonine, de L-méthylthréonine ou de L-alanine.

7. Procédé selon les revendications 1 à 6, caractérisé par le fait qu'on prépare l'ester méthylique de

L-sarcosyl-L-arginyl-L-valyl-L-tyrosyl-L-isoleucyl-L-histidyl-L-prolyl-L-isoleucine,

l'ester méthylique de

L-α-aminooxypropionyl-L-arginyl-L-valyl-L-tyrosyl-L-isoleucyl-L-histidyl-L-prolyl-L-isoleucine,

l'ester méthylique de L-sarcosyl-L-arginyl-L-valyl-L-tyrosyl-L-isoleucyl-L-histidyl-L-prolyl-L-méthylthréonine,

l'ester méthylique d'hydroxyacétyl-L-arginyl-L-valyl-L-tyrosyl-L-isoleucyl-L-histidyl-L-prolyl-L-isoleucine,

l'ester méthylique d'hydroxyacétyl-L-arginyl-L-valyl-L-tyrosyl-L-isoleucyl-L-histidyl-L-prolyl-L-méthylthréonine,

l'ester méthylique d'hydroxyacétyl-L-arginyl-L-valyl-L-tyrosyl-L-isoleucyl-L-histidyl-L-prolyl-L-thréonine

ou l'ester méthylique de L-sarcosyl-L-arginyl-L-valyl-L-tyrosyl-L-isoleucyl-L-histidyl-L-prolyl-L-alanine.

8. Procédé selon les revendications 1 à 7, caractérisé par le fait que par la ou les condensations, on constitue, comme dérivés protégés d'esters de peptide, des dérivés de formule générale:

B-X-Arg(C)-Val-Tyr(D)-Ile-His(E)-Pro-Y-O-A (II)

dans laquelle:

B représente un groupe protecteur séparable par acidolyse ou par hydrogénolyse catalytique, en particulier un groupe benzyloxycarbonyle ou tertiobutyloxycarbonyle,

C représente un groupe protecteur pour la protection temporaire du groupe guanidine du radical de l'arginine, en particulier un groupe nitro ou p-toluènesulfonyle,

D représente un groupe protecteur pour la protection temporaire du groupe hydroxyle aromatique du radical de la tyrosine, en particulier un groupe benzyle éventuellement substitué,

E représente un groupe protecteur pour la protection temporaire du groupe imidazole du radical de l'histidine, en particulier un groupe dinitrophényle, et

X, Arg, Val, Tyr, Ile, His, Pro, Y et A sont tels que définis aux revendications 1 ou 4 à 6.